# EUROPEAN PATENT APPLICATION

(11) **EP 4 445 957 A1**
(43) Date of publication of application: **16.10.2024**
(21) Application number: 23305567.2
(22) Date of filing: 14.04.2023
(51) Int. Cl.: A61P 31/04, C07K 7/06, A61K 38/08

(54) **ANTIBACTERIAL COVALENT PEPTIDE INHIBITORS**

(71) Applicant: Université de Bordeaux, 33000 Bordeaux (FR); Institut Polytechnique de Bordeaux, 33400 Talence (FR); Centre National de la Recherche Scientifique, 75016 Paris (FR)
(72) Inventor: GUICHARD, Gilles, 33170 GRADIGNAN (FR); COMPAIN, Guillaume, 33700 MERIGNAC (FR); BURNOUF, Dominique, 67170 BRUMATH (FR); WAGNER, Jerôme, 67100 STRASBOURG (FR); MONSARRAT, Clément, 33400 TALENCE (FR)
(74) Representative: Lavoix

(57) **Abstract**

The present invention relates to new peptides, in particular as antibacterial agents, and also to therapeutic uses thereof. The present invention also relates to said compounds for use for the treatment of bacterial infections.

This invention relates to the development of antibacterial peptides targeting the histidine residue from Gram-negative bacterial sliding clamp.

## Description

The present invention concerns new covalent peptide inhibitors, in particular as antibacterial agents, and also to therapeutic uses thereof. The present invention also relates to said compounds for use for the treatment of bacterial infections.

This invention relates to the development of antibacterial peptides targeting the histidine residue from Gram-negative and Gram-positive bacterial sliding clamp (His175 in *E. coli* sliding clamp).

The increase in bacterial resistance became a major public health concern over the last decades. It was recently estimated that three million deaths worldwide per year are related to infection to resistant strains. Several Gram-negative bacteria are classified as critical pathogens by WHO, including Enterobacteriaceae, *P*. *aeruginosa* and *A. baumannii.* In this context, it is urgent to develop new antibiotics effective against these pathogens. Bacterial sliding clamp (SC) has been identified as a new target to develop novel antibacterial compounds. This homodimeric protein is used as an anchoring platform for DNA polymerases and plays a pivotal role in DNA replication and metabolism. SC interacts with numerous protein partners through protein-protein interactions (PPIs) at the same well-conserved large and shallow binding site. Notably, when loaded onto DNA, its interaction with replicative DNA polymerases confers to the enzyme a high processivity required for genome duplication and cell multiplication. SC is also used for the recruitment and activities of many proteins involved in DNA metabolism in general, especially in DNA repair as well as in induced and spontaneous mutagenesis. Several research groups have reported molecules able to interact at the protein binding site of the *^{Ec}*SC and inhibit DNA replication (Wijffels, G. et al., Biochemistry 2004, 43 (19), 5661-5671; Georgescu, R. E. et al., Proc. Natl. Acad. Sci. 2008, 105 (32), 11116; Kling, A. et al., Science 2015, 348(6239), 1106-1112; Wolff, P. et al., J. Med. Chem. 2011, 54(13), 4627-4637; Wijffels, G. et al., J. Med. Chem. 2011, 54 (13), 4831-4838; Yin, Z. et al., J. Med. Chem. 2014, 57 (6), 2799-2806; Yin, Z. et al., J. Med. Chem. 2015, 58 (11), 4693-4702; André, C. et al., RSC Chem. Biol. 2020, 1 (3), 137-147; Monsarrat, C. et al., J. Med. Chem. 2021, 64 (23), 17063-17078). The best binding affinities were obtained with short peptides ranging from ~30 to 200 nM. To further improve the potency of these compounds, the inventors envisioned to transform non-covalent inhibitors into targeted covalent inhibitors (TCls). Although there has been a reluctance to apply an irreversible mode of action in drug discovery research for a while, there was recently a resurgence of covalent inhibitors as shown by the late successes of covalent drugs approved over the last ten years (Gehringer, M. et al., J. Med. Chem. 2019, 62 (12), 5673-5724; and Boike et al., Advances in Covalent Drug Discovery. Nat. Rev. Drug Discov. 2022). Covalent inhibitors targeting PPI could endow potential benefits over non-covalent inhibitors (Cheng, S.-S. et al., J. Hematol. Oncol.J Hematol Oncol 2020, 13 (1), 26, and Way, J. et al., Curr. Opin. Chem. Biol. 2000, 4 (1), 40-46). When forming an irreversible bond, the inhibitor is no longer in competition with natural binding proteins. Additionally, covalent inhibition can potentially improve the inhibitory potency and/or selectivity, increase the duration of action and reduce the emergence of resistance. Although some established classes of antibiotics actually work by irreversible inhibition (e.g. β-lactams), the use of TCls to target bacterial machineries through inhibition of PPI still remains overlooked.

To evaluate the possibility to find an effective covalent inhibitor targeting the SC, the inventors started to pinpoint suitable nucleophiles at the protein surface near-by the binding site. His175 from *^{Ec}*SC, a residue ideally located at the central part of the binding site was selected. Notably, this residue is highly conserved among gram-negative and gram-positive species, including numerous medically relevant bacteria (*E*. *coli*, *A. baumanii*, *P. aeruginosa*, *S. aureus*, *E. cloacae*, *S. pyogenes*, *S. pneumoniae*, *Klebsiella pneumonia*, etc.). However, histidine is rarely targeted compared to cysteine or lysine, and remains a challenging target due to its lower nucleophilicity. Yet, histidine is much more frequently found in protein binding sites than cysteine and thus targeting these residues represents a tremendous opportunity. Targeting this amino acid requires i) to fine-tune the geometry of the side chain bearing the weak electrophile to get ideal proximity and relative orientation between the warhead and histidine, ii) to find a good balance of flexibility versus rigidity, and iii) to find the appropriate reactivity of the electrophile.

The aim of the present invention is thus to provide an efficient compound that blocks bacterial DNA synthesis, in particular being able to covalently bind the sliding clamp of bacterial pathogens with high efficiency.

The aim of the present invention is also to provide a new class of antibiotics.

Therefore, the present invention relates to a compound having the following formula (I): 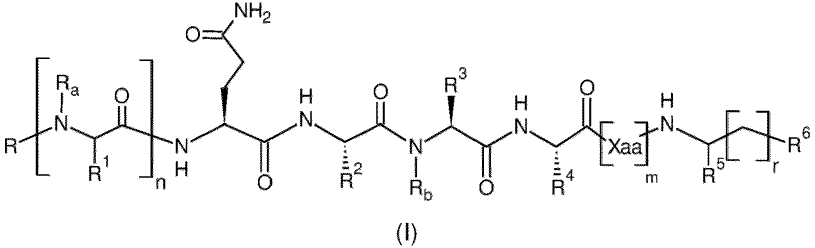 wherein:
- n is 0 or 1;
- m is 0 or 1;
- r is 0 or 1 ;
- R is H or is selected from the group consisting of:
   ∗ a (C₁-C₁₈)alkyl group optionally substituted by a (C₆-C₁₀)aryl group, by a heteroaryl group or by a heterocycloalkyl group,
   ∗ a (C₂-C₁₈)alkenyl group optionally substituted by a (C₆-C₁₀)aryl group,
   ∗ a (C₃-C₆)cycloalkyl group,
   ∗ a (C₆-C₁₀)aryl group optionally substituted by a (C₁-C₄)alkyl,
   ∗ a group having the following formula (II): 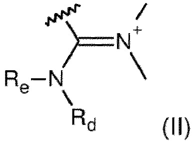 R_{d} and Rₑ representing independently from each other a (C₁-C₆)alkyl group, or forming together with the nitrogen atom carrying them a heterocycloalkyl group, such as a morpholinyl group,
   ∗ a group -C(=O)-R', R' being selected from the group consisting of:
      · a (C₁-C₁₈)alkyl group optionally substituted by an optionally substituted (C₆-C₁₀)aryl group,
      · a -NH-optionally substituted (C₆-C₁₀)aryl group,
      · a (C₃-C₆)cycloalkyl group,
      · a (C₆-C₁₀)aryl group optionally substituted by a (C₁-C₄)alkyl,
- Rₐ, and R_{b} are, independently from each other, selected from the group consisting of: H, and (C₁-C₆)alkyl groups;
- R¹ is H or the side chain of arginine;
- R² is a (C₁-C₆)alkyl group or a -CH₂-(C₃-C₆)cycloalkyl group;
- R³ is selected from the group consisting of one of the following formulae: 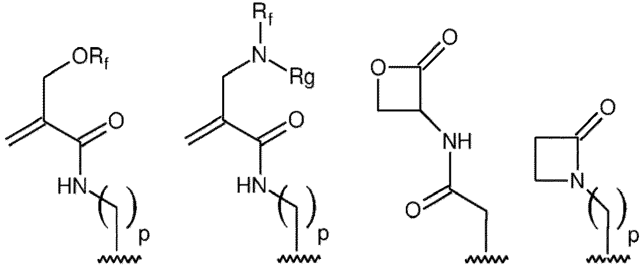 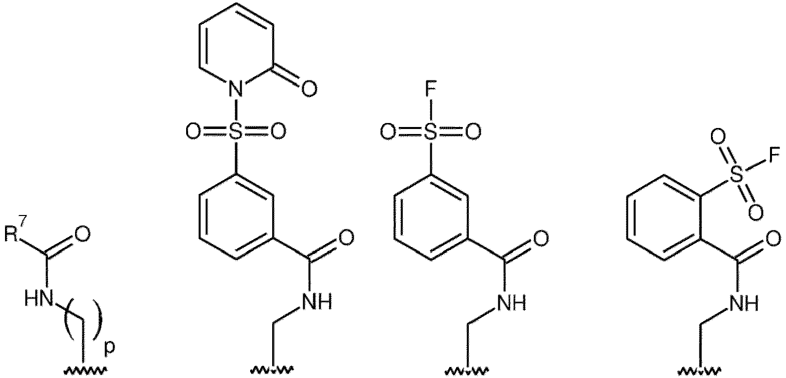 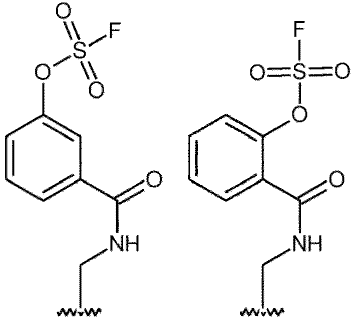 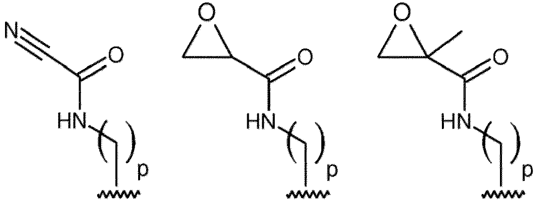 wherein:
   · p is 1, 2 or 3;
   · R_{f} is selected from the group consisting of: (C₁-C₄)alkyl, (C₆-C₁₀)aryl, acetyl, and COORₕ groups, Rₕ being (C₁-C₄)alkyl;
   · R_{g} is selected from the group consisting of: (C₁-C₄)alkyl and (C₆-C₁₀)aryl groups;
   · R⁷ is selected from the group consisting of: -CH₂-X, X being Cl or Br, -CH=CH₂,
   -CH=CH-CH₃, -C≡CH, -C≡C-CH₃, -CH=CH-CH₂-NMe₂, and -CH₂-O-SO₂-NR_{f}R_{g},
- R⁴ is a (C₁-C₈)alkyl group or a (C₁-C₄)alkyl group optionally substituted by at least one halogen or a -CH₂-(C₃-C₆)cycloalkyl group;
- Xaa is selected from the group consisting of:
   · a -N(R_{c})-CH₂-C(=O)- group;
   · a group having the following formula (II-1): 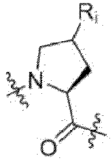
   · a group having the following formula (II-2): 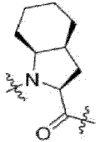 wherein
      R_{c} is H or a (C₁-C₆)alkyl group; and
      Rᵢ is selected from the group consisting of: H, methyl, OH, F, (C₁-C₄)alkoxy group, (C₁-C₄)alkylthio group, -O-benzyl or -S-benzyl;
- R⁵ is selected in the group consisting of:
   ∗ a -(CH₂)-(C₆-C₁₀)aryl group optionally substituted by at least one halogen, (C₁-C₂)alkyl group and/or (C₁-C₂)alkoxy group;
   ∗ a -(CH₂-CH₂)-(C₆-C₁₀)aryl group optionally substituted by at least one halogen, (C₁-C₂)alkyl group and/or (C₁-C₂)alkoxy group;
   ∗ a -(CH₂)-(C₃-C₆)cycloalkyl group;
   ∗ a -(CH₂-CH₂)-(C₃-C₆)cycloalkyl group;
   ∗ a (C₁-C₈)alkyl group, optionally substituted by at least one halogen; and
- R⁶ is H, -COOH, -CONH₂, -COOR¹⁰, -CONHR¹⁰, R¹⁰ being a (C₁-C₈)alkyl group, when r=0; and R⁶ is H, -COOH, - CONH₂, -OH, -NH₂, -COOR¹⁰, -CONHR¹⁰, or -OR¹⁰, when r=1.

The present invention relates to the first covalent inhibitors targeting selectively the His175 residue of *^{Ec}*SC as a representative Gram negative bacteria. This residue is indeed conserved in the sequence of large number of Gram negative bacteria as well as in sequences of some Gram positive bacteria. The inhibitors have been rationally designed based on X-ray structures of peptide-SC complexes recently reported. A series of warheads has been evaluated. X-ray structures of covalent adducts with several TCls were solved at high resolution (< 1.5 Å) and confirmed the covalent bonds with His175. Proteomic analyses demonstrated the high selectivity of the chloroacetamide-based TCI in bacterial cell lysate. Finally, an *in vitro* replication assay reveals the higher inhibitory efficiency of TCI over a non-covalent inhibitor.

As examples of bacteria, the followings may be mentioned: *E. coli*, *K. pneumoniae, A. baumanni, P. aeruginosa, Enterobacter spp, Rickettsia bellii, Rickettsia typhi str. Wilmington, Enterobacter cloacae, Burkholderia cepacia, Legionella pneumophila, Haemophilus influenzae, Bordetella pertussis, Pasteurella, Enterococcus faecium, Neisseria gonorrhoeae, Salmonella bongori, Haemophilus influenzae, Shigella boydii, Rickettsia rickettsii*, *Streptococcus pneumoniae, Caulobacter crescentus*, *Stenotrophomonas maltophilia*, and *Yersinia pestis.*

The following definitions are set forth to illustrate and define the meaning and scope of the various terms used to describe the invention herein.

The expression "Cₜ-C_{z}" means a carbon-based chain that can have from t to z carbon atoms, for example C₁-C₃ means a carbon-based chain that can have from 1 to 3 carbon atoms.

The term "alkyl group" means: a linear or branched, saturated, hydrocarbon-based aliphatic group comprising, unless otherwise mentioned, from 1 to 12 carbon atoms. By way of examples, mention may be made of methyl, ethyl, n-propyl, isopropyl, butyl, isobutyl, tert-butyl or pentyl groups.

The term "aryl group" means: a cyclic aromatic group comprising between 6 and 10 carbon atoms. By way of examples of aryl groups, mention may be made of phenyl or naphthyl groups.

The term "heteroaryl" means: a 5- to 10-membered aromatic monocyclic or bicyclic group containing from 1 to 4 heteroatoms selected from O, S or N. By way of examples, mention may be made of imidazolyl, thiazolyl, oxazolyl, furanyl, thiophenyl, pyrazolyl, oxadiazolyl, tetrazolyl, pyridinyl, pyrazinyl, pyrimidinyl, pyridazinyl, indolyl, benzofuranyl, benzothiophenyl, benzoxazolyl, benzimidazolyl, indazolyl, benzothiazolyl, isobenzothiazolyl, benzotriazolyl, quinolinyl and isoquinolinyl groups.

By way of a heteroaryl comprising 5 to 6 atoms, including 1 to 4 nitrogen atoms, mention may in particular be made of the following representative groups: pyrrolyl, pyrazolyl, 1,2,3-triazolyl, 1,2,4-triazolyl, tetrazolyl and 1,2,3-triazinyl.

Mention may also be made, by way of heteroaryl, of thiophenyl, oxazolyl, furazanyl, 1,2,4-thiadiazolyl, naphthyridinyl, quinoxalinyl, phthalazinyl, imidazo[1,2-a]pyridine, imidazo[2,1-b]thiazolyl, cinnolinyl, benzofurazanyl, azaindolyl, benzimidazolyl, benzothiophenyl, thienopyridyl, thienopyrimidinyl, pyrrolopyridyl, imidazopyridyl, benzoazaindole, 1,2,4-triazinyl, indolizinyl, isoxazolyl, isoquinolinyl, isothiazolyl, purinyl, quinazolinyl, quinolinyl, isoquinolyl, 1,3,4-thiadiazolyl, thiazolyl, isothiazolyl, carbazolyl, and also the corresponding groups resulting from their fusion or from fusion with the phenyl nucleus.

The term "heterocycloalkyl" means: a 4- to 10-membered, saturated or partially unsaturated, monocyclic or bicyclic group comprising from one to three heteroatoms selected from O, S or N; the heterocycloalkyl group may be attached to the rest of the molecule via a carbon atom or via a heteroatom; the term bicyclic heterocycloalkyl includes fused bicycles and spiro-type rings.

By way of saturated heterocycloalkyl comprising from 5 to 6 atoms, mention may be made of oxetanyl, tetrahydrofuranyl, dioxolanyl, pyrrolidinyl, azepinyl, oxazepinyl, pyrazolidinyl, imidazolidinyl, tetrahydrothiophenyl, dithiolanyl, thiazolidinyl, tetrahydropyranyl, tetrahydropyridinyl, dioxanyl, morpholinyl, piperidinyl, piperazinyl, tetrahydrothiopyranyl, dithianyl, thiomorpholinyl or isoxazolidinyl.

Among the heterocycloalkyls, mention may also be made, by way of examples, of bicyclic groups such as (8aR)-hexahydropyrrolo[1,2-a]pyrazin-2(1H)-yl, octahydroindozilinyl, diazepanyl, dihydroimidazopyrazinyl and diazabicycloheptanyl groups, or else diazaspiro rings such as 1,7-diazaspiro[4.4]non-7-yl or 1-ethyl-1,7-diazaspiro[4.4]non-7-yl.

When the heterocycloalkyl is substituted, the substitution(s) may be on one (or more) carbon atom(s) and/or on the heteroatom(s). When the heterocycloalkyl comprises several substituents, they may be borne by one and the same atom or different atoms.

The term "(C₂-C₁₂)alkenyl" refers to a branched or straight-chain monovalent unsaturated aliphatic hydrocarbon group having one or more carbon double bonds, of 2 to 12 (inclusive) carbon atoms, preferably 2 to 8 (inclusive) carbon atoms, more preferably 2 to 4 (inclusive) carbon atoms. This term is further exemplified by groups as vinyl, propylenyl, butenyl, pentenyl, hexenyl, heptenyl, octenyl, nonenyl, decenyl, undecenyl, dodecenyl and their straight-chain and branched and stereo isomers. The "alkenyl" group can optionally be mono-, di-, tri- or multiply-substituted by a halogen and/or a C₆₋₁₀-aryl group, as defined below.

The term "cycloalkyl group" means: a cyclic carbon-based group comprising, unless otherwise mentioned, from 3 to 6 carbon atoms. By way of examples, mention may be made of cyclopropyl, cyclobutyl, cyclopentyl, cyclohexyl, etc. groups.

When an alkyl radical is substituted with an aryl group, the term "arylalkyl" or "aralkyl" radical is used. The "arylalkyl" or "aralkyl" radicals are aryl-alkyl- radicals, the aryl and alkyl groups being as defined above. Among the arylalkyl radicals, mention may in particular be made of the benzyl or phenethyl radicals.

The term "halogen" means: a fluorine, a chlorine, a bromine or an iodine.

The term "alkoxy group" means: an -O-alkyl radical where the alkyl group is as previously defined. By way of examples, mention may be made of -O-(C₁-C₄)alkyl groups, and in particular the -O-methyl group, the -O-ethyl group as -O-Csalkyl group, the -O-propyl group, the -O-isopropyl group, and as -O-C₄alkyl group, the -O-butyl, -O-isobutyl or -O-tert-butyl group.

The abovementioned "alkyl", "cycloalkyl", "aryl", "heteroaryl" and "heterocycloalkyl" radicals can be substituted with one or more substituents. Among these substituents, mention may be made of the following groups: amino, hydroxyl, thiol, oxo, halogen, alkyl, alkoxy, alkylthio, alkylamino, aryloxy, arylalkoxy, cyano, trifluoromethyl, carboxy or carboxyalkyl.

The term "alkylthio" means: an -S-alkyl group, the alkyl group being as defined above.

The term "alkylamino" means: an -NH-alkyl group, the alkyl group being as defined above.

The term "aryloxy" means: an -O-aryl group, the aryl group being as defined above.

The term "arylalkoxy" means: an aryl-alkoxy- group, the aryl and alkoxy groups being as defined above.

The term "carboxyalkyl" means: an HOOC-alkyl- group, the alkyl group being as defined above. As examples of carboxyalkyl groups, mention may in particular be made of carboxymethyl or carboxyethyl.

The term "acetyl" means: "C(=O)CH₃".

The term "carboxyl" means: a COOH group.

The term "oxo" means: "=O".

In some embodiments of the invention, the compounds of the invention contain more than one asymmetric center and thus occur as racemates and racemic mixtures, single enantiomers, individual diastereoisomeric mixtures. All such isomeric forms of these compounds are included in the present invention, unless expressly provided otherwise.

For example, in formula (I), the carbon atom carrying the R¹ group is an asymmetric carbon that has either a configuration R or a configuration S.

For example, in formula (I), the carbon atom carrying the R⁵ group is an asymmetric carbon that has either a configuration R or a configuration S.

In some embodiments, the compounds of the invention contain one or more double bonds and thus occur as individual or mixtures of Z and/or E isomers. All such isomeric forms of these compounds are included in the present invention, unless expressly provided otherwise.

In the embodiments where the compounds of the invention contain multiple tautomeric forms, the present invention also includes all tautomeric forms of said compounds unless expressly provided otherwise.

In some embodiments of the invention, the compounds of the invention are in the form of salts. They can be in non-ionized or in ionized forms.

According to an embodiment, in formula (I), n=0.

According to an embodiment, in formula (I), m=0.

According to an embodiment, in formula (I), r=0.

According to an embodiment, in formula (I), n=0, m=0, and r=0. This specific family consists of compounds having the following formula (I-1): 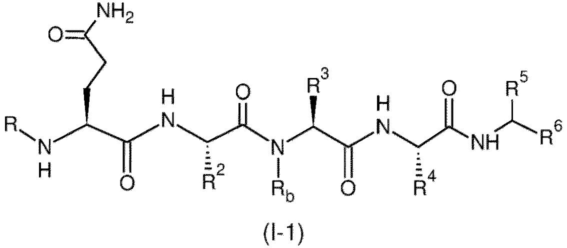 wherein R, R², R³, R_{b}, R⁴, R⁵, and R⁶ are as defined above in formula (I).

According to an embodiment, in formula (I), or in formula (I-1) as defined above, R is selected in the group consisting of:
∗ a (C₁-C₁₈)alkyl group optionally substituted by a (C₆-C₁₀)aryl group, by a heteroaryl group or by a heterocycloalkyl group,
∗ a group having the following formula (II): 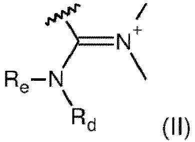 R_{d} and Rₑ representing independently from each other a (C₁-C₆)alkyl group, or forming together with the nitrogen atom carrying them a heterocycloalkyl group, such as a morpholinyl group, and
∗ a group -C(=O)-R', R' being selected in the group consisting of:
   · a (C₁-C₁₈)alkyl group optionally substituted by an optionally substituted (C₆-C₁₀)aryl group, and
   · a -NH-optionally substituted (C₆-C₁₀)aryl group.

According to an embodiment, in formula (I), or in formula (I-1) as defined above, R is a group -C(=O)-R', R' being selected in the group consisting of:
· a (C₁-C₁₈)alkyl group optionally substituted by an optionally substituted (C₆-C₁₀)aryl group, and
· a -NH-optionally substituted (C₆-C₁₀)aryl group.

According to an embodiment, in formula (I), or in formula (I-1) as defined above, R is a group -C(=O)-R', R' being a (C₁-C₁₈)alkyl group optionally substituted by an optionally substituted (C₆-C₁₀)aryl group.

According to an embodiment, in formula (I), or in formula (I-1) as defined above, R is a group -C(=O)-R', R' being a (C₁-C₁₈)alkyl group, preferably a methyl group.

A preferred group of compounds according to the invention consists of compounds of formula (I), or of compounds of formula (I-1) as defined above, wherein R is a -C(=O)-Me group.

According to an embodiment, a specific group of compounds according to the invention consists of compounds having the following formula (I-2): 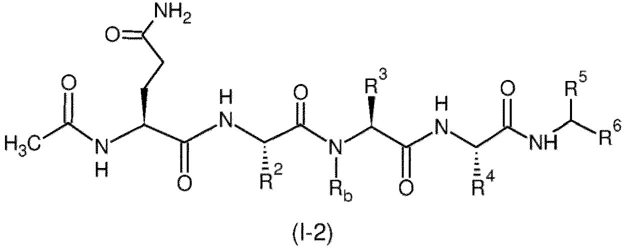 wherein R², R³, R_{b}, R⁴, R⁵, and R⁶ are as defined above in formula (I).

According to an embodiment, in formula (I), or in formula (I-1) as defined above, R² is a -CH₂-(C₃-C₆)cycloalkyl group, preferably a -CH₂-cyclohexyl group.

A preferred group of compounds according to the invention consists of compounds of formula (I), or of compounds of formula (I-1) as defined above, wherein R² is a -CH₂-(C₃-C₆)cycloalkyl group, preferably a -CH₂-cyclohexyl group.

Another preferred group of compounds according to the invention consists of compounds of formula (I-2) as defined above, wherein R² is a -CH₂-(C₃-C₆)cycloalkyl group, preferably a -CH₂-cyclohexyl group.

According to an embodiment, a specific group of compounds according to the invention consists of compounds having the following formula (I-3): 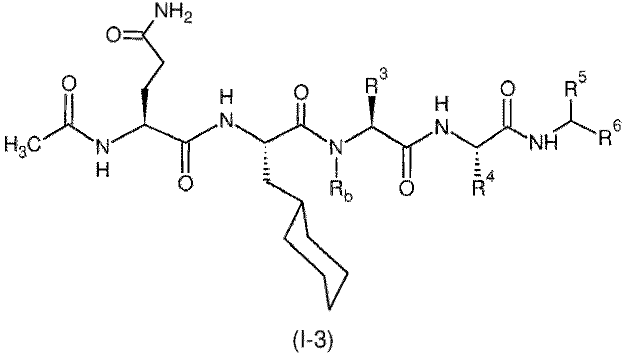 wherein R³, R_{b}, R⁴, R⁵, and R⁶ are as defined above in formula (I).

According to an embodiment, in formula (I), or in formula (I-1), R_{b} is H.

According to an embodiment, a specific group of compounds according to the invention consists of compounds having the following formula (I-4): 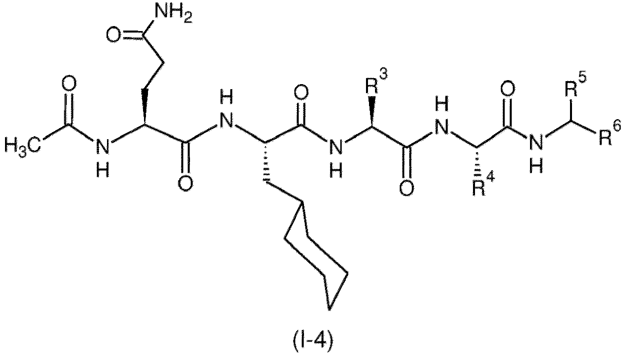 wherein R³, R⁴, R⁵, and R⁶ are as defined above in formula (I).

According to an embodiment, in formula (I), or in any one of formulae (I-1), (I-2), (I-3) or (I-4), R³ is selected from the group consisting of one of the following formulae: 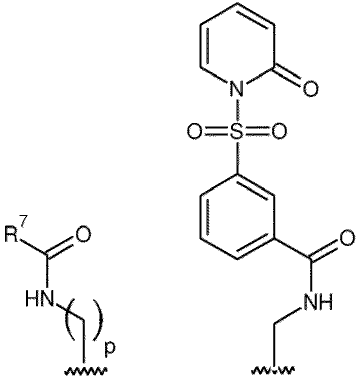 wherein:
· p is 1, 2 or 3, p being preferably 1; and
· R⁷ is selected from the group consisting of: -CH₂-X, X being Cl or Br, -CH=CH₂,
-CH=CH-CH₃, and -C≡C-CH₃.

According to an embodiment, in formula (I), or in any one of formulae (I-1), (I-2), (I-3) or (I-4), R³ has the following formula: 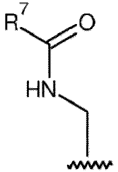 wherein R⁷ is selected from the group consisting of: -CH₂-X, X being Cl or Br, -CH=CH₂, -CH=CH-CH₃, and -C≡C-CH₃.

According to an embodiment, in formula (I), or in any one of formulae (I-1), (I-2), (I-3) or (I-4), R⁴ represents the side chain of leucine, hexafluoroleucine or pentafluoroleucine.

According to an embodiment, in formula (I), or in any one of formulae (I-1), (I-2), (I-3) or (I-4), R⁴ is a (C₁-C₈)alkyl group. More preferably, R⁴ is an isobutyl group.

According to an embodiment, a specific group of compounds according to the invention consists of compounds having the following formula (I-5): 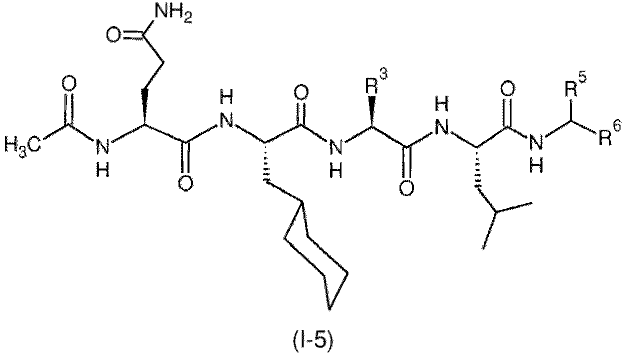 wherein R³, R⁵, and R⁶ are as defined above in formula (I).

According to an embodiment, in formula (I), or in any one of formulae (I-1), (I-2), (I-3), (I-4) or (I-5), R⁵ is a -(CH₂)-(C₆-C₁₀)aryl group optionally substituted by at least one halogen, (C₁-C₂)alkyl group and/or (C₁-C₂)alkoxy group, or a (C₁-C₈)alkyl group or a (C₁-C₄)alkyl group optionally substituted by at least one halogen.

According to an embodiment, in formula (I), or in any one of formulae (I-1), (I-2), (I-3), (I-4) or (I-5), R⁵ is a -(CH₂)-(C₆-C₁₀)aryl group optionally substituted by at least one halogen.

According to an embodiment, in formula (I), or in any one of formulae (I-1), (I-2), (I-3), (I-4) or (I-5), R⁵ is a -(CH₂)-(C₆-C₁₀)aryl group, optionally substituted by at least one halogen, R⁵ being in particular a benzyl group, optionally substituted by at least one halogen, preferably by at least two halogen atoms, such as chlorine.

According to an embodiment, in formula (I), or in any one of formulae (I-1), (I-2), (I-3), (I-4) or (I-5), R⁵ is a benzyl group, or a benzyl group substituted with two chlorine atoms.

According to an embodiment, in formula (I), when r=0, R⁶ is H, -COOH, -CONH₂, or -CO₂R¹⁰, R¹⁰ being a (C₁-C₈)alkyl group, preferably Me.

According to an embodiment, in formula (I), when r=1, R⁶ is H, -COOH, -CONH₂, -OH, -NH₂, or -CO₂R¹⁰, R¹⁰ being a (C₁-C₈)alkyl group, preferably Me.

According to an embodiment, in any one of formulae (I-1), (I-2), (I-3), (I-4) or (I-5), R⁶ is H, -COOH, -CONH₂, or -CO₂R¹⁰, R¹⁰ being a (C₁-C₈)alkyl group, preferably Me.

According to an embodiment, in formula (I), or in any one of formulae (I-1), (I-2), (I-3), (I-4) or (I-5), R⁶ is -COOH.

According to an embodiment, a specific group of compounds according to the invention consists of compounds having the following formula (I-6): 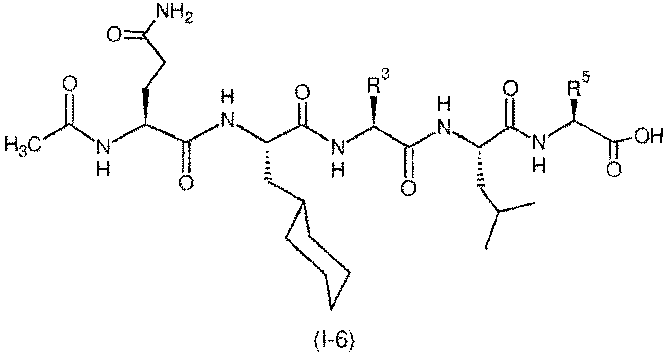 wherein R³ and R⁵ are as defined above in formula (I).

According to an embodiment, a specific group of compounds according to the invention consists of compounds having the following formula (I-7) and (I-8): 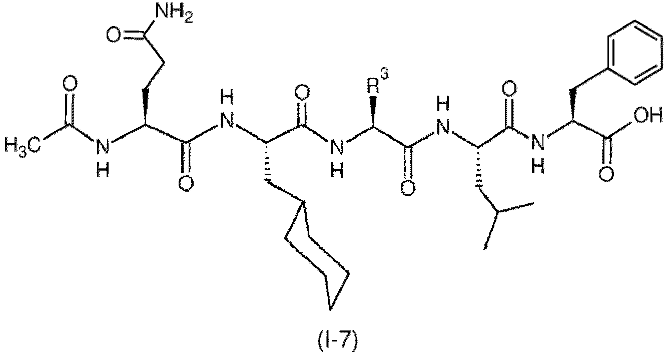 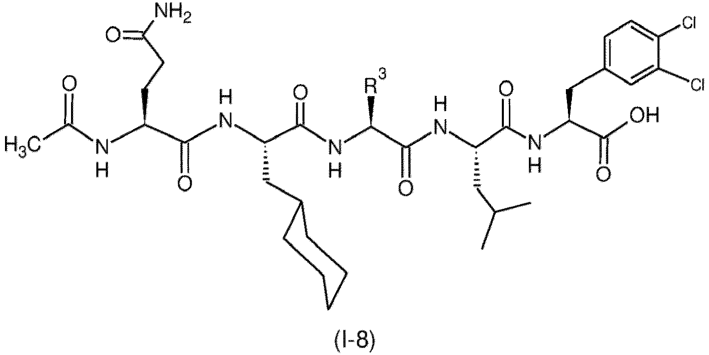 wherein R³ is as defined above in formula (I).

The present invention also relates to the following preferred compounds:

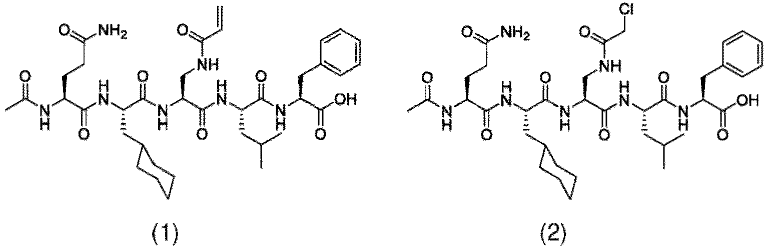

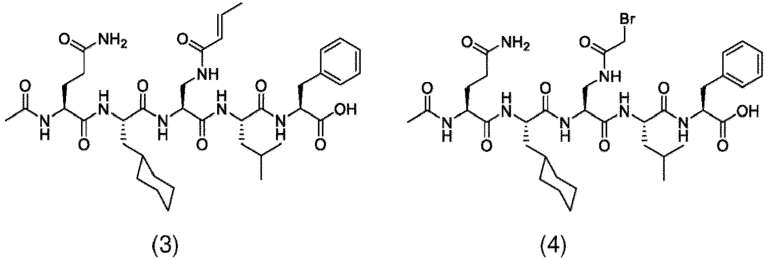

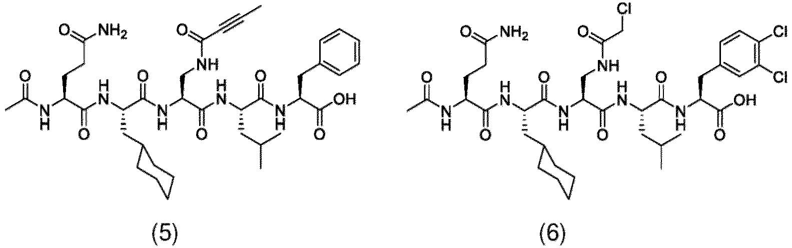

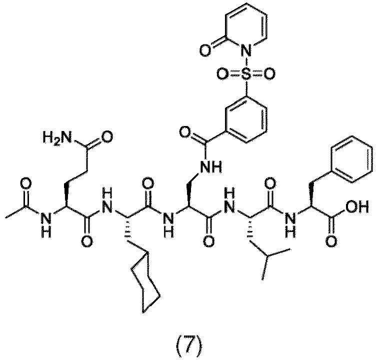

The present invention also relates to a medicament comprising a compound as defined above, preferably a compound of formula (I) as defined above, or a compound having one of the above formulae (I-1), (I-2), (I-3), (I-4), (I-5), (I-6), (I-7) or (I-8).

The present invention also relates to a pharmaceutical composition comprising a compound as defined above, preferably a compound of formula (I) as defined above, or a compound having one of the above formulae (I-1), (I-2), (I-3), (I-4), (I-5), (I-6), (I-7) or (I-8).

While it is possible for the compounds of the invention to be administered alone it is preferred to present them as pharmaceutical compositions. The pharmaceutical compositions, both for veterinary and for human use, useful according to the present invention comprise at least one compound having formula (I), (I-1), (I-2), (I-3), (I-4), (I-5), (I-6), (I-7) or (I-8) as above defined, together with one or more pharmaceutically acceptable carriers and optionally other therapeutic ingredients.

In certain preferred embodiments, active ingredients necessary in combination therapy may be combined in a single pharmaceutical composition for simultaneous administration.

As used herein, the term "pharmaceutically acceptable" and grammatical variations thereof, as they refer to compositions, carriers, diluents and reagents, are used interchangeably and represent that the materials are capable of administration to or upon a mammal without the production of undesirable physiological effects such as nausea, dizziness, gastric upset and the like.

The preparation of a pharmacological composition that contains active ingredients dissolved or dispersed therein is well understood in the art and need not be limited based on formulation. Typically, such compositions are prepared as injectables either as liquid solutions or suspensions; however, solid forms suitable for solution, or suspensions, in liquid prior to use can also be prepared. The preparation can also be emulsified. In particular, the pharmaceutical compositions may be formulated in solid dosage form, for example capsules, tablets, pills, powders, dragees or granules.

The choice of vehicle and the content of active substance in the vehicle are generally determined in accordance with the solubility and chemical properties of the active compound, the particular mode of administration and the provisions to be observed in pharmaceutical practice. For example, excipients such as lactose, sodium citrate, calcium carbonate, dicalcium phosphate and disintegrating agents such as starch, alginic acids and certain complex silicates combined with lubricants such as magnesium stearate, sodium lauryl sulphate and talc may be used for preparing tablets. To prepare a capsule, it is advantageous to use lactose and high molecular weight polyethylene glycols. When aqueous suspensions are used they can contain emulsifying agents or agents which facilitate suspension. Diluents such as sucrose, ethanol, polyethylene glycol, propylene glycol, glycerol and chloroform or mixtures thereof may also be used.

The pharmaceutical compositions can be administered in a suitable formulation to humans and animals by topical or systemic administration, including oral, rectal, nasal, buccal, ocular, sublingual, transdermal, topical, vaginal, parenteral (including subcutaneous, intra-arterial, intramuscular, intravenous, intradermal, intrathecal and epidural), intracisternal and intraperitoneal. It will be appreciated that the preferred route may vary with for example the condition of the recipient.

The formulations can be prepared in unit dosage form by any of the methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then, if necessary, shaping the product.

Total daily dose of the compounds of the invention administered to a subject in single or divided doses may be in amounts, for example, of from about 0.001 to about 100 mg/kg body weight daily and preferably 0.01 to 10 mg/kg/day. Dosage unit compositions may contain such amounts of such submultiples thereof as may be used to make up the daily dose. It will be understood, however, that the specific dose level for any particular patient will depend upon a variety of factors including the body weight, general health, sex, diet, time and route of administration, rates of absorption and excretion, combination with other drugs and the severity of the particular disease being treated.

The present invention also relates to the compound of formula (I), (I-1), (I-2), (I-3), (I-4), (I-5), (I-6), (I-7) or (I-8) as defined above for use as an antibacterial agent.

The present invention also relates to a compound of formula (I), (I-1), (I-2), (I-3), (I-4), (I-5), (I-6), (I-7) or (I-8) as defined above for use for the treatment of bacterial infections.

According to an embodiment, the bacterial infections are caused by Gram negative and Gram-positive pathogens. Preferably, said bacterial infections are caused by Gram-negative bacteria, including those belonging to the following bacteria orders: Enterobacterales, Pseudomonales, Burkholderiales, Neisseriales, Campylobacterales, and Thiotrichales.

According to an embodiment, said bacterial infections are caused by bacteria selected from the group consisting of: bacteria from the genus *Escherichia* sp, in particular *E*. *coli;* bacteria from the genus *Klebsiella* sp, in particular *K. pneumoniae*; bacteria from the genus *Shigella* sp., in particular *S*. *sonnei*, S. *dysenteriae*, and *S*. *flexneri*; bacteria from the genus *Salmonella* sp., in particular *S*. *enterica*, *S. typhi*, and *S*. *parathyphi*; and bacteria from the genus *Yersinia* sp., in particular *Y. enterocolitica* and *Y. pestis.*

According to an embodiment, said bacterial infections are caused by bacteria selected from the group consisting of: bacteria from the genus *Acinetobacter* sp, in particular *A.baumannii*; and bacteria from the genus *Pseudomonas* sp., in particular *P. aeruginosa.*

According to an embodiment, said bacterial infections are caused by bacteria selected from the group consisting of: bacteria from the genus *Burkholderia* sp, in particular *Burkholderia cepacia*, *Burkholderia mallei*, and *Burkholderia pseudomallei.*

According to an embodiment, said bacterial infections are caused by bacteria selected from the group consisting of: bacteria from the genus *Neisseria* sp, in particular *N.gonorrhoeae* and *N. meningitidis.*

According to an embodiment, said bacterial infections are caused by bacteria selected from the group consisting of: bacteria from the genus *Campylobacter* sp, in particular *C. jejuni.*

According to an embodiment, said bacterial infections are caused by bacteria selected from the group consisting of: bacteria from the genus *Francisella* sp., in particular *Francisella tularensis.*

According to an embodiment, the infections according to the invention are chosen from infections caused by multi-drug resistant (MDR) bacteria, extensively drug-resistant (XDR) bacteria or pandrug-resistant (PDR) bacteria, derived from the above bacteria.

According to an embodiment, the infections according to the invention are chosen from infections caused by antibiotic-resistant bacteria, in particular with carbepenem resistance, cephalosporin resistance, or fluoroquinolone resistance. Such antibiotic-resistant bacteria are in particular mentioned in the WHO website (https://www.who.int/news/item/27-02-2017-who-publishes-list-of-bacteria-for-which- new-antibiotics-are-urgently-needed).

According to an embodiment, the bacterial infections are selected from the group consisting of the following infections: respiratory infections, stomach infections, gastrointestinal infections, blood infections, skin infections, bladder infections, kidney infections, urinary tract infections, ear infections, eye infections, and meningeal infections. A skin infection may include an infection of a mucosal membrane, such as the oral cavity, esophagus or eye, e.g. cornea.

In the context of the invention, the term "treating" or "treatment", as used herein, means reversing, alleviating, inhibiting the progress of, or preventing the disorder or condition to which such term applies, or one or more symptoms of such disorder or condition.

### FIGURE DESCRIPTION

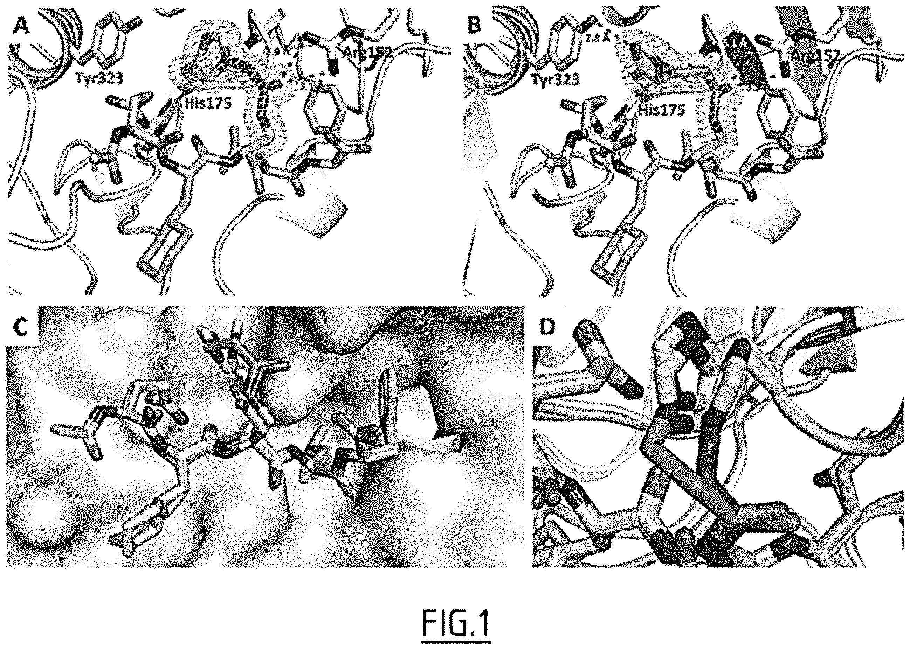

**Figure 1****.** Crystal structures of *^{Ec}*SC-**2** adduct (**A**) solved at 1.21 Å resolution and *^{Ec}*SC-**1** adduct (**B**) solved at 1.43 Å resolution. The electron density is shown as grid around the covalent bond formed with His175. (**C**) Superimposition of crystal structures of *^{Ec}*SC-**12** complex, *^{Ec}*SC-**2** (RMSD of atomic positions with *^{Ec}*SC-**12** complex = 0.498 Å (316 to 316 atoms)) and *^{Ec}*SC-**1** (RMSD of atomic positions with *^{Ec}*SC-**12** = 0.461 Å (334 to 334 atoms)) adducts showing that the canonical binding mode of the peptide **12** is fully conserved on covalent adducts with **2** and **1**. (D) Zoom on the covalent bonds showing the rotation of His175 on *^{Ec}*SC-**2** compared to *^{Ec}*SC-**12** and *^{Ec}*SC-**1** crystal structures.

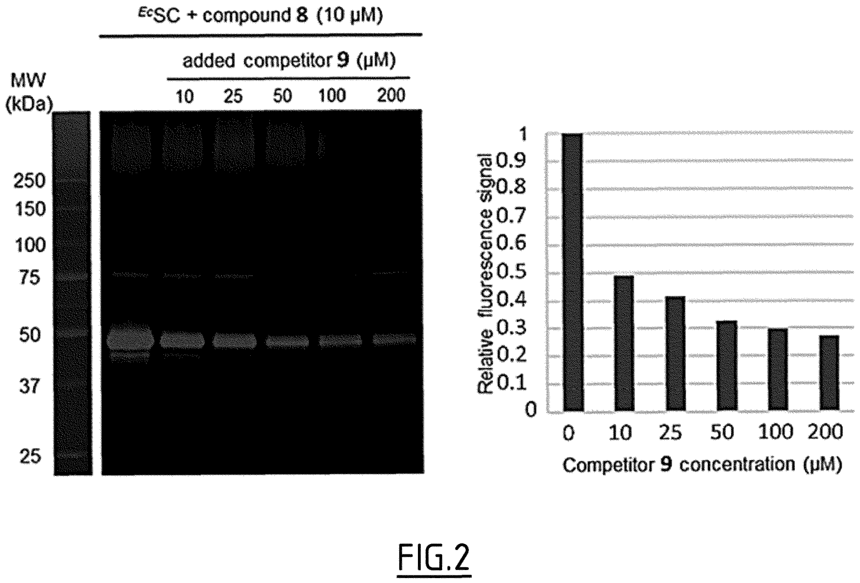

**Figure 2****. The chloroacetamide-based TCI 8 outcompetes a higher affinity non-covalent binder (9).**
   Upper part: 2 µM of purified SC is incubated with a fixed amount of **8** (10 µM) and increasing concentrations of the non-covalent but high-affinity SC-binder **9** as indicated. After 1 hour incubation at 37 °C and SDS-PAGE separation of the reaction products, biotinylated SC-bound TCI were revealed by in-gel detection using streptavidin coupled to an IRDye. Lower part: relative fluorescence detected in each lane is plotted against the non-covalent competitor concentration. Quantification shows that even a 50-fold excess of non-covalent SC-binder do not outcompete the TCI.

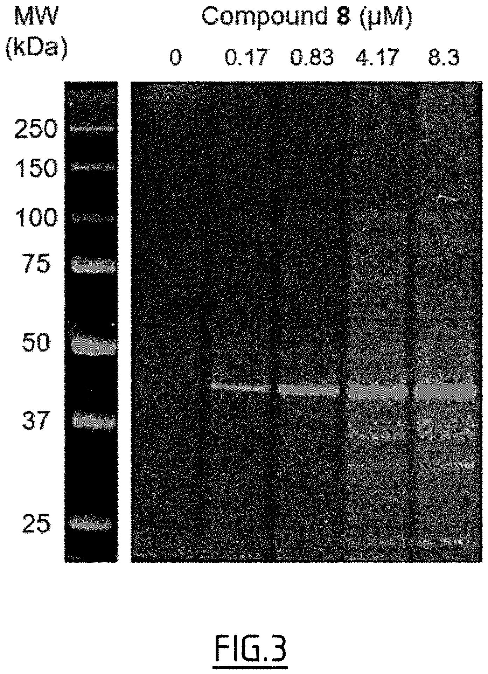

**Figure 3****. The chloroacetamide-based TCI 8 reacts predominantly with one protein in *E. coli* whole cell extract.**
   1 mg of bacterial whole cell extract is incubated with increasing amounts of biotinylated compound **8** as indicated. Biotinylated peptides and reaction products are captured onto streptavidin coated magnetic beads and analysed by SDS-PAGE. Only biotinylated products are visualized by in-gel detection using streptavidin coupled to an IRDye and near-infrared imaging. The MW of the most intense band detected (around 40-45 kDa) indicates that the major reaction product formed most probably represents the covalent **8**-SC adduct.

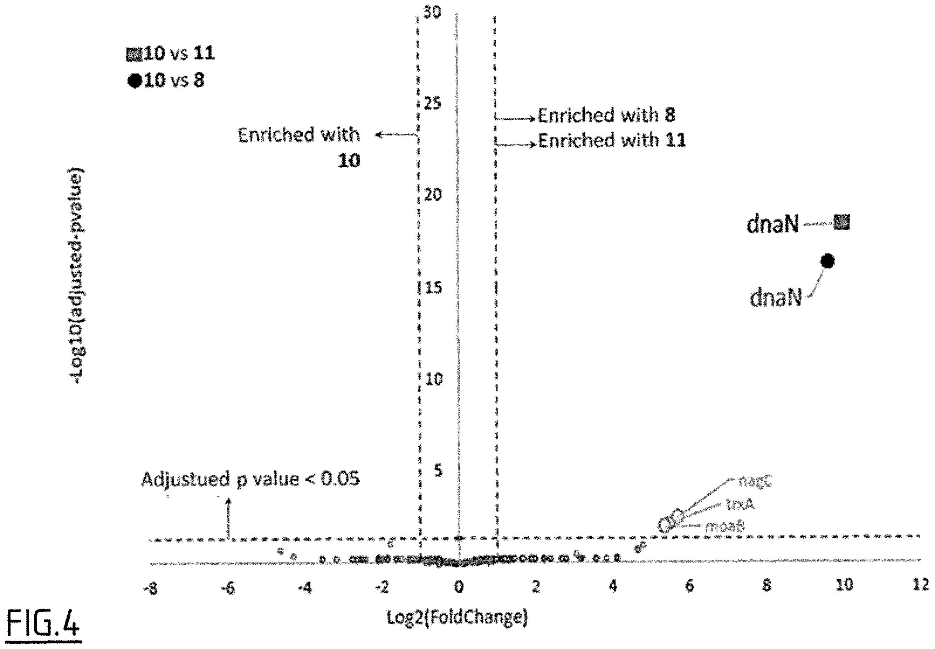

**Figure 4****. The SC is specifically engaged by compounds 8 and 11 in *E. coli* whole cell lysates.**
   Proteomic analysis of pull-down experiments in whole cell lysate. The volcano plot displays the log2 fold change (x axis) against the -log10 adjusted p-value (y axis) for all proteins enriched when using peptide 10 (control, left part of the graph) compared with SC-binding peptides 8 (circles) or 11 (squares). Cut-offs (dashed lines) of log2FC > 1 (ratio > 2) and -log10 adj p value > 1.3 (adjusted p value < 0.05) were applied to highlight significantly enriched proteins.

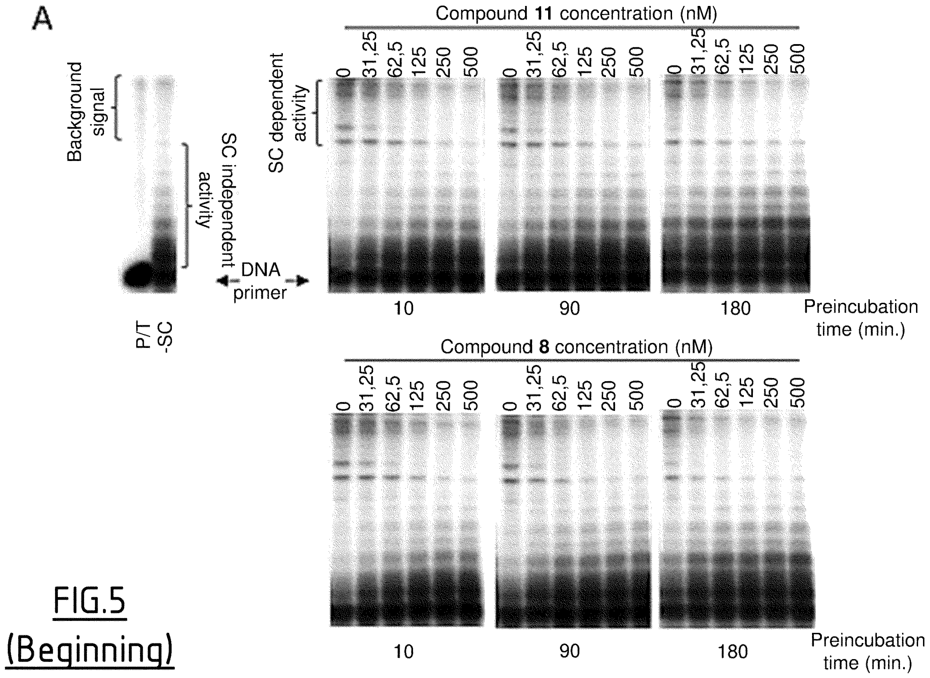

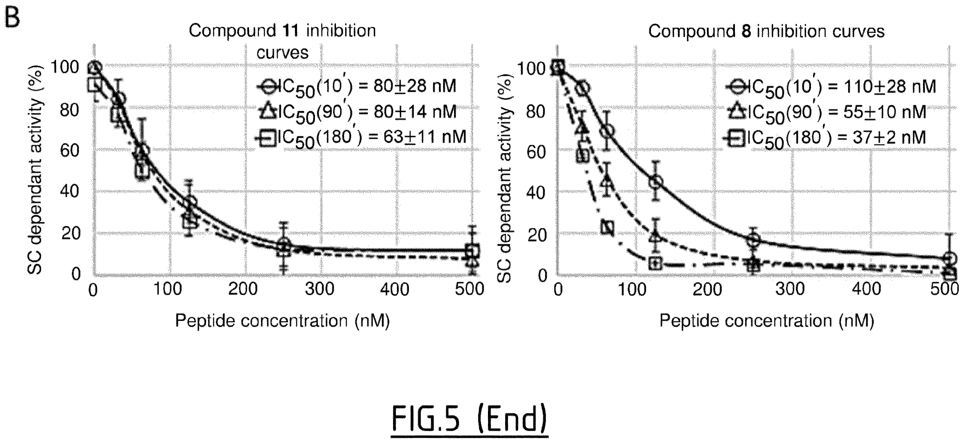

**Figure 5****. The chloroacetamide-based TCI shows superior inhibitory potency over non-covalent inhibitor.**
   Efficiency of the non-covalent (**11**) and covalent (**8**) SC binders to inhibit SC dependent DNA synthesis *in vitro.* (**A**) Typical images of the analysis of DNA synthesis inhibition assays. Upper left part, signals obtained when labelled DNA substrate only (P/T) or the reaction products of the complete assay but the SC (-SC control) are loaded. (**B**) Quantitative analysis of the inhibition efficiency of the two SC binders. Plots of the percentage of SC dependent DNA synthesis as a function of peptide concentration are shown for each condition: 10 min. (circles, solid lines), 90 min. (triangles, dotted lines) or 180 min. (squares, dashed lines) preincubation of SC with the peptides. Each point and error bars represent the average and standard deviations (sd) of two independent assays. These plots were used to determine the indicated IC₅₀ ± sd.

### EXAMPLES

### PEPTIDE SYNTHESIS

### General methods for peptide synthesis

Commercially available reagents were used throughout without purification. Resins and *N*-Fmoc amino acids (Fmoc-Phe-OH, Fmoc-Leu-OH, Fmoc-Gln(Trt)-OH, Fmoc-Cha-OH, Fmoc-Asp(tBu)-OH, Fmoc-Arg(Pbf)-OH, Fmoc-Gly-OH, Fmoc-Pro-OH, Fmoc-Oic-OH, Fmoc-Phe(3,4-Cl₂)-OH, Fmoc-Dap(alloc)-OH, Fmoc-Lys(biot)-OH) were purchased from PolyPeptide Laboratories France, Sigma-Aldrich, Fluorochem, Novabiochem, and Iris Biotech. The coupling agents were purchased from Sigma-Aldrich and Fluorochem. Solid-phase peptide synthesis grade organic solvents (DMF and DCM) were used for solid-phase synthesis and were purchased from Carlo Erba.

Peptides were synthesized by solid-phase methodology in a stepwise manner using standard *N*-(9-fluorenyl)methoxycarbonyl (Fmoc) chemistry on an automated synthesizer (Liberty blue) or manually with µwave assistance (CEM DiscoverBio instrument). Assembly of the protected peptide chains was carried out on a 0.1 mmole or 0.05 mmole starting from either Fmoc-Phe Wang resin (peptides **1-5**). Prior to the synthesis, the resins were swollen for 15 min in a CH₂Cl₂/DMF mixture in polypropylene syringes equipped with a Teflon filter.

Reverse-phase high-performance liquid chromatography (RP-HPLC), quality acetonitrile (ACN) and Milli-Q water were used for RP-HPLC analyses and purification. RP-HPLC analyses were performed on a Dionex U3000SD using a Macherey-Nagel Nucleodur column (4.6 × 100 mm, 3 µm) at a flow rate of 1 mL.min⁻¹. The mobile phase was composed of 0.1% (v/v) trifluoroacetic acid (TFA) in Milli-Q water (solvent A) and 0.1% TFA in ACN (solvent B). The detection was performed at 200 nm and the column temperature in an oven was 25 °C. Semi-preparative purifications of peptides were performed on a Gilson GX-281 system using a Macherey-Nagel Nucleodure column (20 × 250 mm, 5 µm) at a flow rate of 20 mL.min⁻¹. The mobile phase was similar as for the analytical system, unless otherwise notified. A gradient elution (0-20 min: 20 to 60% B) was applied at a flow rate of 20 mL.min⁻¹. Column effluent was monitored by UV detection at 200 nm. The purity of the analyzed compounds was determined to be ≥95% by using the data processing application of Chromoleon7 software.

Liquid chromatography-mass spectrometry (LC-MS) analyses were carried out on an Agilent G6230B TOF spectrometer coupled with an Agilent HPLC 1290 Infinity.

### Attachment of the first amino acid on resin (peptide 6)

The Wang resin was swollen in a syringe for 15 min in DCM. In parallel, 6 equiv of Fmoc amino acid (relative to the resin) were dissolved in DCM. The solution was cooled to 0°C under argon. 5 equiv of DIC (relative to the resin) were added to the amino acid solution to prepare the symmetric anhydride. The mixture was stirred for 20 min at 0 °C. The solvent was evaporated under reduced pressure. The residue was dissolved in a minimum of DMF (~ 3 mL).

The solvent of the resin was removed and 0.1 equiv of DMAP (relative to the resin) was added in the syringe. The solution of anhydride was added to the syringe containing the resin. The suspension was shaken overnight at room temperature. The solution was removed from the syringe and the resin was washed with DMF and DCM.

### Peptide elongation

### Procedure for manual synthesis under micro-waves irradiation (peptides 1-5)

### • Fmoc deprotection

4 mL of a solution of piperidine 20% (v/v) in DMF were added in the vessel containing the swollen resin. The suspension was irradiated under 50 W for 5 min at 75 °C. The solution was removed from the vessel and the resin was washed with DMF (x2) and DCM (x2). The deprotection step was repeated three times. After the third deprotection, a Kaiser test or chloranil was performed.

### • Amino acid coupling

A solution of Oxyma pure (6 equiv. relative to the resin loading in 2 mL of DMF) was prepared. 3 equiv. of Fmoc-amino acid were dissolved in 2 mL of the Oxyma pure solution. Then, 3 equiv. of DIC were added. The solution was then added in the vessel containing the swollen resin. The suspension was irradiated under 30 W for 10 min at 75 °C. The solution was removed from the vessel and the resin was washed with DMF (x2) and DCM (x2). The coupling step was repeated twice. At the end of the second coupling, a Kaiser test (primary amine) or chloranil test (secondary amine) was performed.

### Procedure for automatic synthesis under micro-waves irradiation (peptides 6 and 7)

The resin was placed into a reaction vessel and was allowed to swell using DCM for 30 min. After swelling, the peptides were synthesized using an automated synthesizer with microwave assistance by repetiting of the following steps:
1) The Fmoc deprotection was performed twice with a solution of 20% (v/v) piperidine in DMF: 75 °C, 155 W, 15 s, then at 90 °C, 35 W, 50 s.
2) The coupling reactions were performed with Fmoc amino acid (0.2 M in DMF), DIC (0.5 M in DMF) in the presence of Oxyma pure (1 M in DMF). The *L*- and D-arginine couplings were performed twice at 25 °C, 0 W, 1500 s with 6 equiv. of Fmoc-Arg(Pbf)-OH and 6 equiv. of coupling agents for the first coupling, and 10 equiv. of Fmoc-Arg(Pbf)-OH and 10 equiv. of coupling agents for the second coupling (A. C. Seefeldt, F. Nguyen, S. Antunes, N. Pérébaskine, M. Graf, S. Arenz, K. K. Inampudi, C. Douat, G. Guichard, D. N. Wilson, C. A. Innis, Nat. Struct. Mol. Biol. 2015, 22, 470-475). Other amino acid couplings were performed first at 90 °C, 170 W, 115 s, then at 90 °C, 30 W, 110 s with 6 equiv. of Fmoc-Xaa-OH and 6 equiv. of coupling agents. The resin was washed with 5 mL of DMF (x3) between each step.

### Acetylation at the N-terminal position

### • Automatic synthesis under micro-waves irradiation (peptides 1-5)

The acetylation was performed with acetic acid (0.2 M solution of DMF), DIC (0.5 M in DMF) in presence of Oxyma pure (1 M in DMF). The reaction was performed first at 90 °C, 170 W, 115 s, then at 90 °C, 30 W, 110 s with 6 equiv. of Fmoc-Xaa-OH and 6 equiv. of coupling agents. The resin was washed with 5 mL of DMF (x3).

### Functionalization of Dap side chain

### Chemioselective Alloc deprotection (peptides 1-5)

The swollen resin containing the peptide with the *N*-Alloc protected amino acid in the syringe, was placed under argon. 0.1 equiv. of Pd(PPh₃)₄ was added to the resin. A solution of 10 equiv. of PhSiH₃ in 3 mL in DCM was added into the syringe. The suspension was stirred for 20 min at room temperature. The solution was removed from the syringe and the resin was washed with DMF (x2) and DCM (x2). The deprotection by palladium was repeated thrice and the Kaiser test was performed.

### Coupling of bromoacetyl bromide (Peptide 4)

After the selective Alloc deprotection of Dap(Alloc), a solution of 10 equiv. of TEA in 3 mL of DCM was prepared. Then, 5 equiv. of bromoacetyl bromide were added. This solution was added in the syringe containing the swollen resin. The suspension was stirred for 1 h at room temperature. The solution was removed from the syringe and the resin was washed with DMF (x2) and DCM (x2). The reaction was performed thrice and a Kaiser test was performed.

### Coupling of chloroacetyl chloride (Peptide 2)

After the selective Alloc deprotection of Dap(Alloc), a solution of 10 equiv. of TEA in 3 mL of DCM was prepared. Then, 5 equiv. of chloroacetyl chloride were added. This solution was added in the syringe containing the swollen resin. The suspension was stirred for 1 h at room temperature. The solution was removed from the syringe and the resin was washed with DMF (x2) and DCM (x2). The reaction was performed thrice. A Kaiser test was performed.

### Coupling of acryloyl chloride (Peptide 1)

After the selective Alloc deprotection of the Dap(Alloc) residue, a solution of 10 equiv. of TEA in 3 mL of DCM was prepared. Then, 5 equiv. of acryloyl chloride were added. This solution was added in the syringe containing the swollen resin. The suspension was stirred for 1 h at room temperature. The solution was removed from the syringe and the resin was washed with DMF (x2) and DCM (x2). The reaction was performed twice. A Kaiser test was performed.

### Coupling of crotonic acid (Peptide 3)

After selective Alloc deprotection of Dap(Alloc), a solution of Oxyma pure (4 equiv. relative to the resin loading in 2 mL of DMF) was prepared. 4 equiv. of crotonic acid were dissolved in 2 mL of the solution of DMF. Then, 4 equiv. of DIC were added. The solution was added in the vessel containing the swollen resin. The suspension was irradiated under 30 W during 10 min at 75 °C. The solution was removed from the vessel and the resin was washed DMF (x2) and DCM (x2). The coupling reaction was repeated twice. At the end of the second coupling reaction, a Kaiser test was performed.

### Coupling of 2-butynoic acid (Peptide 5)

After the selective Alloc deprotection of Dap(Alloc), a solution of Oxyma pure (4 equiv. relative to the resin loading in 2 mL of DMF) was prepared. 4 equiv. of 2-butynoic acid were dissolved in 2 mL of the solution of DMF. Then, 4 equiv. of DIC were added. The solution was then added in the vessel containing the swollen resin. The suspension was irradiated under 30 W during x2) and DCM (x2). The reaction was performed twice and a Kaiser test was performed.

### General procedure for the cleavage of the peptides from the resin (peptides 1-5)

At the end of the elongation of the peptide chain, the resin was washed with diethylether or CH₂Cl₂ and dried under high vacuum. Then, the cleavage mixture TFA/H₂O/TIS (95:2.5:2.5; 4 mL) was added to the resin. The mixture was gently shaken for 1-2 h [or 4 h in the presence of Arg(Pbf)], and the resulting solution was flushed through a frit. The cleavage cocktail was removed under reduced pressure and the residue was dissolved directly in a mixture of H₂O/CH₃CN and freeze-dried.

### Analyses and purification of peptides (peptides 1-5)

The crude peptide was analyzed by HPLC (using a H₂O with 0.1% TFA/ CH₃CN with 0.1% TFA mixture, gradient 90/10 - 0/100 for 10 min, 25 °C) and by LCMS (H₂O with 0.1% TFA/ CH₃CN with 0.1% TFA, gradient from 90/10 to 0/100 or 70/30 - 0/100 for 5 min, 40 °C) and purified on semi-preparative HPLC. Then, the pure fractions were combined and freeze-dried. Salt exchange from trifluoroacetate to chloride was performed by solubilizing the peptide in a mixture of CH₃CN and aqueous HCl solution (0.1 M) followed by freeze-drying. The dry solid was characterized by HPLC and LCMS. Finally, the solid was dissolved in CH₃CN and H₂O and the solution was divided in several Eppendorf vials. The aliquot solutions in vials were freeze-dried.

### Peptide 4

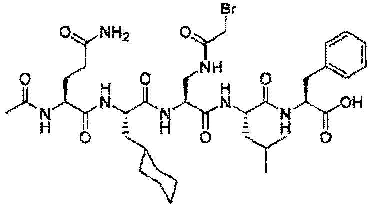

Ac-Gln-Cha-Dap(bromoacetamide)-Leu-Phe-OH
M = 807.32 g.mol⁻¹
Synthesis from 0.1 mmol scale with Wang resin (loading = 0.52 mmol/g)
Purification gradient: 35-55% ACN (0,1% TFA) at 25 °C for 15 min; mₚᵤᵣₑ = 41.4 mg (51% yield)
**MS (ESI+):** (m/z) = 810.47 [M+H]⁺.

### Peptide 2

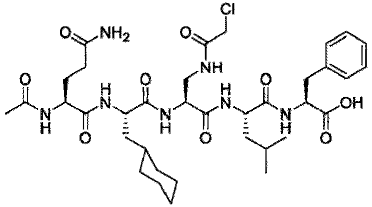

Ac-Gln-Cha-Dap(chloroacetamide)-Leu-Phe-OH
M = 763.37 g.mol⁻¹
Synthesis from 0.1 mmol scale with Fmoc-Phe preloaded Wang resin (loading = 0.52 mmol/g)
Purification gradient: 35 to 55% B in 15 min; mₚᵤᵣₑ = 12.5 mg (16% yield)
**MS (ESI+):** (m/z) = 764.44 [M+H]⁺.

### Peptide 1

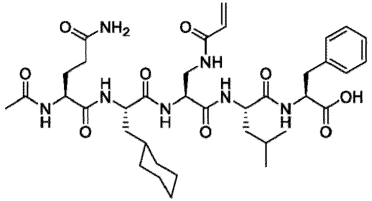

Ac-Gln-Cha-Dap(acrylamide)-Leu-Phe-OH
M = 741.89 g.mol⁻¹
Synthesis from 0.1 mmol scale with Fmoc-Phe preloaded Wang resin (loading = 0.52 mmol/g)
Purification gradient: 35 to 55% B in 15 min; mₚᵤᵣₑ = 22.7 mg (24% yield)
**MS (ESI+):** (m/z) = 742.25 [M+H]⁺; 1483.57 [2M+H]⁺.

### Peptide 3

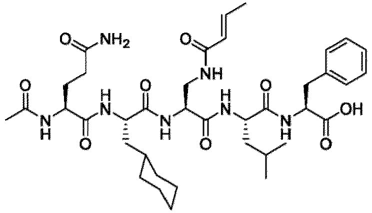

Ac-Gln-Cha-Dap(but-2-enamide)-Leu-Phe-OH
M = 755.91 g.mol⁻¹
Synthesis from 0.1 mmol scale with Fmoc-Phe preloaded Wang resin (loading = 0.52 mmol/g)
Purification gradient: 35 to 55% B in 15 min; mₚᵤᵣₑ = 38.3 mg (51% yield)
**MS (ESI+):** (m/z) = 756.61 [M+H]⁺; 1512.09 [2M+H]⁺.

### Peptide 5

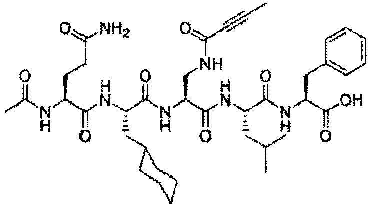

Ac-Gln-Cha-Dap(but-2-ynamide)-Leu-Phe-OH
M = 753.90 g.mol⁻¹
Synthesis from 0.1 mmol scale with Fmoc-Phe preloaded Wang resin (loading = 0.52 mmol/g)
Purification gradient: 35 to 55% B in 15 min; mₚᵤᵣₑ = 33.1 mg (35% yield)
**MS (ESI+):** (m/z) = 754.25 [M+H]⁺; 1529.55 [2M+H]⁺.

### Peptide 6

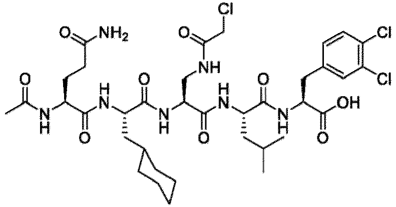

Ac-Gln-Cha-Dap(chloroacetamide)-Leu-diClPhe-OH
M = 833.20 g.mol⁻¹
Synthesis from 0.1 mmol scale with Wang resin (loading = 0.37 mmol/g)
Purification gradient: 40 to 60% B in 15 min; mₚᵤᵣₑ = 17.4 mg (21% yield)
**MS (ESI+):** (m/z) = 834.42 [M+H]⁺.

### Peptide 7

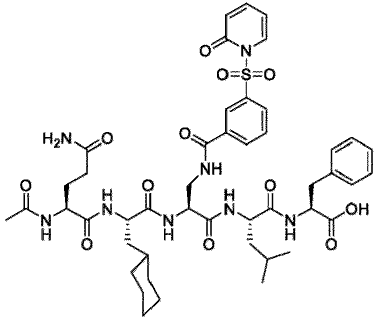

Ac-Gln-Cha-Dap(*N*-sulfonyl pyridone)-Leu-Phe-OH
M = 949.09 g.mol⁻¹
Synthesis from 0.1 mmol scale with Fmoc-Phe preloaded Wang resin (loading = 0.33 mmol/g)
Purification gradient: 40-80% ACN (0,1% TFA) at 25 °C for 15 min; mₚᵤᵣₑ = 8.6 mg (9% yield)
MS (ESI+): (m/z) = 949.42 [M+H]⁺

### THERMODYNAMIC AND KINETIC CONSIDERATIONS FOR THE ^{EC}SC-PEPTIDE INTERACTION

We consider the following two-steps reaction model to describe the binding of the peptide to SC and the covalent bond formation to the His 175 residue:
- First step: the peptides bind reversibly to the SC binding site
- Second: the formation of the covalent bond.

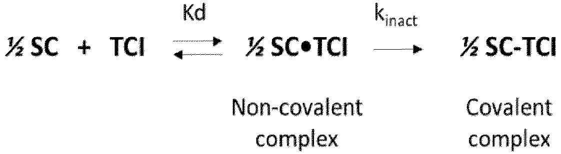

### Isothermal Titration Calorimetry (ITC)

ITC experiments were performed using an iTC200 or a PEAQ-ITC instrument (Microcal Malvern Panalytical). Peptides (300 or 600 µM) were titrated at 25 °C by sequential injections (usually 2 µL each) into an SC solution (30 or 60 µM). Data were corrected for the heat of injection by subtracting the signal of the titration of peptides into protein-free buffer solution either R1 buffer ([4-(2-hydroxyethyl)piperazine-1-ethanesulfonic acid (HEPES) 10 mM pH 7.4, NaCl 0.15 M, EDTA 3 mM] or AcNH4 pH7.4, 150 mM). Each titration was performed at least twice: the exact number of the experiments performed with each peptide is indicated in Tables 1-6. Reported data are mean values (± SD) of all experiments. Analyses of the experimental data were performed, following a classical treatment with AFFINImeter software (https://www.affinimeter.com; S4S, Santiago de Compostela, Spain). In addition, kinetic information was obtained in some cases with kinITC as implemented in software AFFINImeter (D. Burnouf et al., J. Am. Chem. Soc. 2012, 134, 559-565; and P. Dumas et al., in Methods Enzymol., Elsevier, 2016, pp. 157-180). All thermodynamic data are provided in Tables 1-6.

### Peptide 12

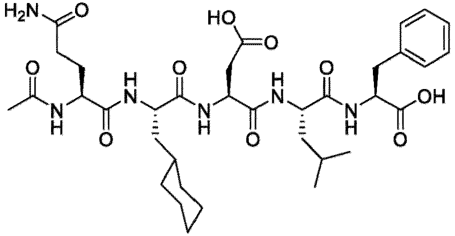

**Table 1. Thermodynamic parameters of the interaction between ^{Ec_{wt}}SC and peptide 12, 1, 2, 3, 4, and 5, in R1 buffer, at 25 °C (298.15K). n: number of experiments.**

| Peptide | K_{D} (nM) | ΔH (kcal/mol) | -TΔS (kcal/mol) | ΔG (kcal/mol) | kₒₙ (M⁻¹.s⁻¹) | k_{off} (s⁻¹) | n |
|---|---|---|---|---|---|---|---|
| **12** | 173 (± 67) | -15.6 (± 1.8) | 6.4 (± 1.7) | -9.3 (± 0.2) | 6.2 (± 3.7) 10⁴ | 9.6 (± 4.4) 10⁻³ | 8 |
| **4** | 141 (± 38) | -12.1 (± 1.4) | 3.0 (± 1.6) | -9.1 (± 0.4) | 5.6 (± 1.3) 10⁴ | 7.5 (± 1.0) 10⁻³ | 5 |
| **2** | 221 (± 80) | -11.7 (± 0.4) | 2.5 (± 0.4) | -9.2 (± 0.1) | 3.6 (± 0.3) 10⁴ | 6.7 (± 1.3) 10⁻³ | 4 |
| **1** | 191 (± 22) | -12.8 (± 0.08) | 3.7 (± 0.02) | -9.2 (± 0.07) | 3.7 (± 1.4) 10⁴ | 7.2 (± 3.3) 10⁻³ | 5 |
| **3** | 166 (± 4) | -10.4 (± 0.1) | 1.1 (± 0.1) | -9.25 (± 0.01) | 2.5 (± 1.9) 10⁴ | 4.2 (± 0.2) 10⁻³ | 2 |
| **5** | 66.4 (± 9.8) | -11.8 (± 0.06) | 2.1 (± 0.1) | -9.8 (± 0.1) | 6.5 (± 4.3) 10⁴ | 4.1 (± 2.2) 10⁻³ | 2 |

**Table 2. Thermodynamic parameters of the interaction between ^{EcH175G}SC and peptides 12, 1, 2, 4, and 5, in R1 buffer, at 25 °C (298.15 K). n: number of experiments.**

| Peptide | K_{D} (µM) | ΔH (kcal/mol) | -TΔS (kcal/mol) | ΔG (kcal/mol) | kₒₙ (M⁻¹.s⁻¹) | k_{off} (s⁻¹) | n |
|---|---|---|---|---|---|---|---|
| **12** | 2.5 (±1.3) | -11.7 (±1.2) | 3.9 (±1.2) | -7.7 (±0.3) | 1.9 (±1.7) 10⁴ | 3.5 (±1.2) 10⁻² | 4 |
| **4** | 0.73 (±0.15) | -8.43 (±0.2) | 0.06 (0.28) | -8.4 (±0.1) | 3.8 (±1.8) 10⁴ | 3.5 (±1.2) 10⁻² | 3 |
| **2** | 0.77 (±0.09) | -9.6 (±0.1) | 1.2 (±0.1) | -8.3 (±0.08) | 1.9 (±0.5) 10⁴ | 1.4 (±0.2) 10⁻² | 3 |
| **1** | 0.77 (±0.12) | -11.6 (±0.6) | 3.3 (±0.7) | -8.3 (±0.1) | 3.0 (±0.4) 10⁴ | 2.3 (±0.3) 10⁻² | 3 |
| **5** | 0.082 (±0.011) | -9.7 (±0.2) | 0.04 (±0.3) | -9.7 (±0.1) | 7.3 (±1.0) 10⁴ | 6.1 (±1.6) 10⁻³ | 3 |

**Table 3. thermodynamic values of the interaction between ^{Ecwt}SC with peptides 12, 1, 2, 3, 4, and 5 in buffer R1 (X 1) at 295.15 K (22 °C). Data in parenthesis are standard deviations. n: number of experiments.**

| Peptide | K_{D} (nM) | ΔH (kcal/mol) | -TΔS (kcal/mol) | ΔG (kcal/mol) | kₒₙ (M⁻¹.s⁻¹) | k_{off} (s⁻¹) | n |
|---|---|---|---|---|---|---|---|
| **12** | 129 (± 15) | -13.6 (± 0.6) | 3.8 (± 0.6) | -9.3 (± 0.1) | 4.6 (± 2.5) 10⁴ | 6.2 (± 4.1) 10⁻³ | 2 |
| **4** | 96.4 (±41) | -11.6 (± 1.1) | 2.1 (± 1.0) | -9.5 (± 0.2) | 3.4 (± 0.5) 10⁴ | 1.2 (± 1.1) 10⁻³ | 3 |
| **2** | 127 (± 6) | -8.9 (± 0.1) | -0.3 (± 0.1) | -9.31 (± 0.03) | 4.3 (± 0.1) 10⁴ | 5.5 (± 0.1) 10⁻³ | 2 |
| **1** | 118 (± 33) | -11.4 (± 0.4) | 2.1 (± 0.2) | -9.4 (± 0.2) | 3.7 (± 0.2) 10⁴ | 5.1 (± 0.4) 10⁻³ | 3 |
| **3** | 66.1 (± 18.2) | -11.7 (± 0.6) | 1.8 (± 0.4) | -9.7 (± 0.2) | 4.6 (± 1.0) 10⁴ | 3.1 (± 1.5) 10⁻³ | 2 |
| **5** | 18.4 (± 5.4) | -13.1 (± 1.2) | 2.7 (± 1.2) | -10.5 (± 0.2) | 9.1 (± 1.2) 10⁴ | 1.6 (± 0.3) 10⁻³ | 3 |

**Table 4. Thermodynamic values of the interaction between ^{Ecwt}SC with peptides 12, 1, 2, 3, and 4 in buffer AcNH₄ pH 7.4, 150 mM, at 295.15 K (22 °C). Data in parenthesis are standard deviations. n: number of experiments.**

| Peptide | K_{D} (nM) | ΔH (kcal/mol) | -TΔS (kcal/mol) | ΔG (kcal/mol) | kₒₙ (M⁻¹.s⁻¹) | k_{off} (s⁻¹) | n |
|---|---|---|---|---|---|---|---|
| **12** | 175 (± 1) | -11.6 (± 0.2) | 2.5 (± 0.2) | -9.1 (± 0.1) | 3.7 (± 1.3) 10⁴ | 6.6 (± 2.3) 10⁻³ | 2 |
| **4** | 111 (± 17) | -13.4 (± 1.0) | 4 (± 1) | -9.4 (± 0.1) | 8.3 (± 5.0) 10⁴ | 9.6 (± 6.4) 10⁻³ | 4 |
| **2** | 146 (± 20) | -10.9 (± 1.5) | 1.7 (± 1.5) | -9.2 (± 0.1) | 5.44 (± 0.03) 10⁴ | 8.1 (± 1.4) 10⁻³ | 3 |
| **1** | 153 (± 84) | -11.5 (± 0.5) | 2.3 (± 0.7) | -9.3 (± 0.3) | 5.8 (± 2.1) 10⁴ | 9.8 (± 9.1) 10⁻³ | 4 |
| **3** | 212 (± 56) | -9.81 (± 0.4) | 0.79 (± 0.52) | -9.02 (± 0.2) | 3.25 (± 0.05) 10⁴ | 6.9 (± 1.7) 10⁻³ | 2 |

**Table 5. Thermodynamic values of the interaction between ^{Ec_{wt}}SC with peptides 12, 1, 2, 3, 4, and 5 in buffer R1 (X 1) at 310.15 K (37 °C). Data in parenthesis are standard deviations. n: number of experiments.**

| Peptide | K_{D} (nM) | ΔH (kcal/mol) | -TΔS (kcal/mol) | ΔG (kcal/mol) | kₒₙ (M⁻¹.s⁻¹) | k_{off} (s⁻¹) | n |
|---|---|---|---|---|---|---|---|
| **12** | 546 (± 12) | -16.9 (± 0.4) | 8.01 (± 0.49) | -8.89 (± 0.01) | 2.8 (± 0.6) 10⁴ | 1.5 (± 2.7) 10⁻² | 2 |
| **4** | 273 (± 37) | -18.4 (± 2.9) | 9.1 (± 2.9) | -9.32 (± 0.08) | 4.6 (± 0.4) 10⁴ | 1.2 (± 0.2) 10⁻² | 5 |
| **2** | 452 (± 183) | -11.3 (± 0.8) | 2.3 (± 0.9) | -9.03 (± 0.23) | 2.7 (± 0.2) 10⁴ | 1.3 (± 0.5) 10⁻² | 3 |
| **1** | 446 (± 163) | -13.9 (± 0.8) | 4.9 (± 1.1) | -9.04 (± 0.26) | 3.1 (± 0.3) 10⁴ | 1.7 (±0.3) 10⁻² | 2 |
| **3** | 402 (± 113) | -14.8 (± 0.8) | 5.8 (± 1) | -9.1 (± 0.2) | 4.0 (± 1.9) 10⁴ | 1.5 (± 0.3) 10⁻² | 2 |
| **5** | 74 (± 3) | -17.0 (± 0.4) | 6.9 (± 0.4) | -10.12 (± 0.03) | 5.9 (± 0.7) 10⁴ | 4.4 (± 0.7) 10⁻² | 2 |

**Table 6. Thermodynamic values of the interaction between ^{Ecwt}SC with peptides 12, 1, 2, 3, and 4 in buffer AcNH₄ pH 7.4, 150 mM, at 310.15 K (37 °C). Data in parenthesis are standard deviations. n: number of experiments.**

| Peptide | K_{D} (nM) | ΔH (kcal/mol) | -TΔS (kcal/mol) | ΔG (kcal/mol) | kₒₙ (M⁻¹.s⁻¹) | k_{off} (s⁻¹) | n |
|---|---|---|---|---|---|---|---|
| **12** | 486 (± 22) | -14.6 (± 0.1) | 5.7 (± 0.1) | -8.96 (± 0.02) | 3.3 (± 0.3) 10⁴ | 1.61 (± 0.06) 10⁻² | 2 |
| **4** | 281 (± 62) | -19.6 (± 0.6) | 10.3 (± 0.7) | -9.3 (± 0.1) | 5.9 (± 2.5) 10⁴ | 1.7 (± 1) 10⁻² | 3 |
| **2** | 580 (± 315) | -16.9 (± 2.7) | 8.1 (± 3.0) | -8.9 (± 0.4) | 7.4 (± 2.0) 10⁴ | 3.7 (± 2.1) 10⁻² | 3 |
| **1** | 482 (± 89) | -15.9 (± 1.2) | 6.9 (± 1.1) | -8.9 (± 0.1) | 5.4 (± 1.4) 10⁴ | 2.5 (± 0.4) 10⁻² | 3 |
| **3** | 475 (± 46) | -15.3 (± 0.1) | 6.31 (±0.02) | -8.97 (± 0.06) | 3.9 (± 0.9) 10⁴ | 1.9 (± 0.6) 10⁻² | 2 |

Comparison of the thermodynamic profiles describing the interaction between ^{*Ec*wt}SC and the various peptides indicates that they all bind to the clamp according to the same enthalpy-driven mechanism. Moreover, they all interact as efficiently as **12** with ^{*Ec*_{wt}}SC. The control peptide **12** presents a larger enthalpy variation, probably because of its capability to form a H-bond between the D3 residue and the *^{Ec}*SC H175 residue. Indeed, this value is reduced (+ 3.9 kcal/mol) when using the H175G SC mutant, with a noticeable effect on the ΔG value (+ 1.5 kcal/mol), linked to a threefold increase in the k_{off} value, a threefold reduction of the kₒₙ value and a resulting K_{D} that is 14-fold lower with ^{*Ec*_{H175}}SC (Table 1 and Table 2). A reduction in ΔH values is also observed for the interaction of peptides **1, 2, 3, 4** and **5,** in which the Asp3 residue is changed into different warheads, with ^{*Ec*_{wt}}SC (Table 1). This suggests that the carboxyl group of the reactive side chain is not involved in the interaction with the H175 residue. Nevertheless, the ΔG values are almost not affected, as compared to **12,** indicating that the various chemical modifications somehow compensate the loss of the hydrogen bond and have major effect on the peptide binding rate constants to ^{*Ec*_{wt}}SC (Table 1), except for peptide **5.** However, we note that the ΔH values vary significantly, depending on the nature of the warhead. This could be related to a differential entropic effect linked to the hydrophobic character of the side chain modification, as exemplified by the reduction of the entropic factor (Δ(-TΔS) = - 2.6 kcal/mol) between **1** (acrylamide group) and **3** (crotonamide group).

The peptides binding onto ^{*Ec*_{H175G}}SC follows the same interaction mode as with ^{*Ec*_{wt}}SC, but with a 4-fold reduction of the affinity, except for **5** (Table 2). This decrease in affinity is essentially due to an increase of the k_{off} values (Table 2). This underlined the fact that the H175 residue somehow contributes to stabilize these peptides, even in absence of a H-bond, as observed for the **1**-^{*Ec*_{wt}}SC complex (Table 1). As compared to the interaction with ^{*Ec*_{wt}}SC, a ΔH reduction is observed for peptides **2**, **1** and **5** (ΔΔH = 3.2, 2.1 and 2.0 kcal /mol, respectively). It is partly related to the concomitant reduction of the entropic factors that could be attributed to a better hydrophobic interaction of the modified chain within the pocket resulting from the H175G mutation. In particular, for **5**, the unfavorable entropic factor is totally abolished (Table 2).

We have characterized the interaction of all peptides (**12** as a control) at 22 and 37 °C, in R1 and in AcNH₄ buffers by ITC (Tables 3 - 6). In all conditions, the interaction remains an enthalpy-driven process. In terms of affinity, there is no drastic difference between the two buffers at a given temperature. As compared to 22 °C, we observed a three-fold decrease in affinity at 37°C, as expected with the increase in temperature, due to a higher k_{off} value. All together, these data indicate that the SC/peptide interaction in AcNH₄ buffer is not different from that observed in R1 buffer and validate the experimental conditions chosen for MS analyses.

### RESULTS

### Structure-based design of peptide covalent inhibitors targeting His175

To assess the feasibility of this approach, peptide SC TCls were designed based on the X-ray co-crystal structure of peptide ligand **1** bound to *^{Ec}*SC that we previously reported (André, C.; Martiel, I.; Wolff, P.; Landolfo, M.; Lorber, B.; Silva da Veiga, C.; Dejaegere, A.; Dumas, P.; Guichard, G.; Oliéric, V.; Wagner, J.; Burnouf, D. Y. Interaction of a Model Peptide on Gram Negative and Gram Positive Bacterial Sliding Clamps. ACS Infect. Dis. 2019, 5 (6), 1022-1034). The imidazole group of His175 is oriented towards the side chain of the peptide's central amino acid, Asp3, interacting together through a hydrogen bond (HB). The oxygen of the carboxylic acid is distant from the nitrogen of the imidazole by 2.9 Å, and thus His175 residue is ideally located. Hence, we considered replacing Asp3 of peptide **12** with a Dap residue to graft different warheads with a gradual variation of reactivity and with different geometries and rigidities. These weak electrophiles are all being used in approved drugs or drug candidates under clinical trials. The electrophiles were incorporated onto the solid support, at the last step of the synthesis, after the orthogonal removal of the alloc group on the Dap residue.

### The chloroacetamide-based TCI efficiently binds to His175

### Determination of thermodynamic and kinetic constants

Next, the ability of these compounds to bind and react with the protein was evaluated according to the general reaction scheme as mentioned above. The inventors determined the affinity of TCls by Isothermal Titration Calorimetry (ITC) to assess the influence of the modification at the Asp3 residue on the binding dissociation constants K_{D} at 25 °C as well as at 22 °C and 37 °C (Table 7, and above Tables 1 and 3-6).

**Table 7. Dissociation constants and second-order rate constants k_{inact} of compounds 12 and 1-5.**

| **Entry** | **Compound** | **12** | **4** | **2** | **1** | **3** | **5** |
|---|---|---|---|---|---|---|---|
| 1 | K_{D} (nM) at 25°C (Hepes buffer) | 173 ±67 | 139 ± 54 | 221 ±80 | 191 ±22 | 172 ±10 | 66.4 ±9.8 |
| 2 | K_{D} (nM) at 22 °C (Hepes buffer) | 129 ±15 | 96 ± 41 | 127 ±6 | 118 ±33 | 66 ± 10 | 18 ± 5 |
| 3 | K_{D} (nM) at 22 °C (AcONH₄ buffer) | 175 ±1 | 111 ± 17 | 146 ±20 | 153 ±84 | 212 ± 56 | n.d. |
| 4 | k_{inact} (M⁻¹. s⁻¹) at 22 °C (AcONH₄ buffer) | - | n.d. | 0.193 ±3 10⁻³ | 0.058 ±4 10⁻³ | n.d. | n.d. |
| 5 | K_{D} (nM) at 37 °C (Hepes buffer) | 546 ±12 | 273 ± 36 | 452 ±183 | 446 ±163 | 402 ± 113 | 74 ± 3 |
| 6 | K_{D} (nM) at 37 °C (AcONH₄ buffer) | 486 ±22 | 281 ± 62 | 580 ±315 | 482 ±89 | 475 ±47 | n.d. |
| 7 | k_{inact} (M⁻¹. s⁻¹) at 37 °C (AcONH₄ buffer) | - | n.d. | 1.04 ±0.01 | 0,024 ±6 10⁻³. | n.d. | n.d. |

Overall, no major effect of the incorporation of the warheads on the binding affinity was observed on the whole series of the TCls. Derivatives **1** to **4** bind to SC with an affinity similar to the one of non-covalent inhibitor **12** (173 nM at 25 °C), whereas the introduction a 2-butynamide with a linear carbon chain (compound **5**) led to a significant increase in affinity (66 nM at 25 °C, Table 1). Then, monitoring the reaction of compounds **1, 2** and **4** by mass spectrometry revealed that these compounds form covalent adducts with *^{Ec}*SC. Then, kinetic constants (k_{inact}) of compounds **1** and **2** were determined at 22°C and 37°C (Table 7, entries 4 and 7).

### X-ray crystal structure of covalent adducts shows the covalent bond with His175

To confirm the formation of the covalent bond with His175, the inventors crystalized adducts formed between *^{Ec}*SC and compounds **1** and **2**, which both gave the promising kinetic results. We solved the structure obtained with **2** at an excellent resolution of 1.21 Å (Fig. 1A). The structure unambiguously revealed the covalent bond with His175 as shown by the electron density at the N-C bond. Interestingly, the carbonyl group of the acetamide moiety is involved in a bidentate HB with Arg152. This additional hydrogen bond could contribute positively to the kinetic of the covalent bond formation by controlling the conformation and by activating the electrophilic carbon. Furthermore, we also solved a 1.37-Å crystal structure of **1** bound to *^{Ec}*SC (Fig. 1B). The electron density clearly shows the covalent bond with His175. Peptides **2** and **1** superimpose very well with peptide **12** in complex with *^{Ec}*SC (RMSD: 0.498 Å (316 to 316 atoms) and 0.461 Å (334 to 334 atoms), respectively) (Fig. 1C), showing that the covalent bond does not affect the structure of the complex, in good agreement with dissociation constants discussed above. Interestingly, His175 is slightly rotated on the *^{Ec}*SC-**2** structure compared to *^{Ec}*SC-**12** complex and *^{Ec}*SC-**1**, presumably to better accommodate the shorted side chain with the chloroacetamide (peptide **2**) compared to peptide **1** containing an acrylamide group (Fig. 1D).

### The chloroacetamide-based TCI outcompetes non-covalent binders

To show the ability of the TCI to compete with non-covalent SC binders, competitive experiments were performed in a concentration-dependent manner with biotinylated derivative **8** and high-affinity non-covalent inhibitor **9** previously reported (K_{D} = 39 nM)( Monsarrat, C.; Compain, G.; André, C.; Engilberge, S.; Martiel, I.; Oliéric, V.; Wolff, P.; Brillet, K.; Landolfo, M.; Silva da Veiga, C.; Wagner, J.; Guichard, G.; Burnouf, D. Y. Iterative Structure-Based Optimization of Short Peptides Targeting the Bacterial Sliding Clamp. J. Med. Chem. 2021, 64 (23), 17063-17078). 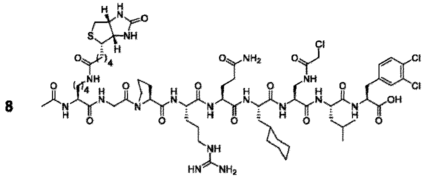 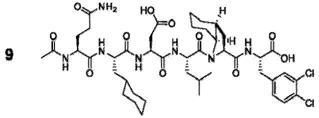 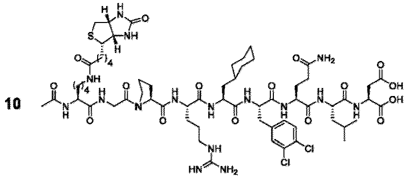 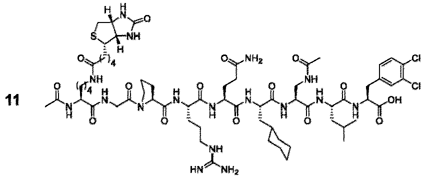 Several aliquots containing purified recombinant His-tagged *^{Ec}*SC were incubated for 1 hour with a fixed peptide **8** concentration and an increasing amount of **9**, and covalent products were analyzed under denaturing conditions (Fig. 2). An intense band appeared at around 40 kDa at a concentration of 10 µM of **8** and was attributed to the covalent adduct formed with *^{Ec}*SC. When increasing the concentration of competitor **9** from 0 to 200 µM), the intensity of the band decreased up to -40% and reached a plateau. These results clearly indicate that both compounds compete for the same binding site on the protein and that chloroacetamide-based TCI **8** outcompetes the non-covalent inhibitor.

### The chloroacetamide-based TCI reacts selectively and covalently to SC in E. coli whole cell lysate

Then, the inventors evaluated the selectivity of the chloroacetamide-based TCI **8** and its ability to covalently bind to *^{Ec}*SC in *E. coli* whole cell lysate. First, cell lysates (1 mg proteins) were treated with different concentrations of **8** ranging from 0.17 to 8.3 µM for 1 hour at 37 °C. Biotinylated molecules were enriched using streptavidin coated magnetic beads, separated by denaturing SDS-PAGE and visualized by near-infrared imaging of a IRDye modified Streptavidin directly infused in the gel (Fig. 3). The intensity of a major band at about 40-45 kDa, compatible with the molecular weight of *^{Ec}*SC monomer (40.6 kDa), increases with the concentration of **8** until 4.17 µM) showing the concentration dependency of the alkylation of this particular protein. Other peptide-protein complexes are clearly observed at high peptide concentrations (4.17 µM) but the intensities of these do not increase when the TCI concentration is doubled and thus may be indicative of non-specific binding.

In order to directly address the selectivity of **8**, a set of pull-down experiments was performed with biological triplicates to detect by mass spectrometry which proteins are interacting with either the biotinylated TCI **8**, its non-covalent counterpart **11** or the non-SC-binding peptide control **10** (Scramble peptide). The total number of MS/MS fragmentation spectra matching on all identified *E. coli* proteins in all replicates (Spectral Count quantification) were submitted to a negative-binomial statistical test in R to highlight the statistically significant enriched proteins, as previously described (Kuhn, L.; Vincent, T.; Hammann, P.; Zuber, H. Exploring Protein Interactome Data with IPinquiry: Statistical Analysis and Data Visualization by Spectral Counts. In Statistical Analysis of Proteomic Data; Burger, T., Ed.; Methods in Molecular Biology; Springer US: New York, NY, 2023; Vol. 2426, pp 243-265).

A total of 24 peptides were identified on SC sequence by Mascot, one of the most widely used database search engine in proteomics. As the biotinylated peptide **8** used for the pull-down experiment can be cleaved by trypsin during the enzymatic digestion, a variable modification was included during the database search, corresponding to a mass increment of +753.302 Da due the covalent binding of the last five residues of compound **8** (C₃₄H₄₉N₇O₈Cl₂). Moreover, 2 other algorithms (Sequest and MS-Amanda), in combination with the Percolator tool, were also used to strongly validate the identification of the covalently modified SC peptide. This multiple search engines strategy allows us to validate the detection of the C-terminal five last residues of **8** covalently bound to the histidine residue of the SC peptide TVATDGHR. Importantly, Percolator tool allowed us to consider all the different fragment series and returned a q-value of 1.0 e⁻³. The fragmentation from the peptide confirmed without any ambiguity the presence of consecutive *y* and *y** fragment ions that precisely diagnose the localization of the modification on the histidine residue. Moreover, the detection of both *y* and *y** fragment ions (regular *y* ions and their respective amine neutral loss fragments) reinforces the validation of the modification because the amine-part of the modification is a glutamine residue. Interestingly, a careful comparison of the isotopic pattern from both precursors with their theoretical isotopic profiles (through IsoPro3.0 simulator) was helpful to highlight a distortion of the isotopic pattern from the histidine-modified peptide towards the third isotope (n+2). This gives an additional clue to confirm the presence of chlore in the modification of SC by the TCI, as the 2 main isotopes of chlore are ³⁵Cl and ³⁷Cl.

To further estimate the proportion of the histidine-modified peptide TVATDGHR compared to the non-modified peptide, an accurate MS1 label-free quantification was carried out in Proteome Discoverer package. The elution peaks from the 24 peptides matching on SC sequence were reconstructed in the 4 conditions (total *E. coli* lysate, scrambled peptide, non-covalent peptide, covalent peptide) and their average intensities in the 3 biological replicates were compared. As expected, the mass increment of +753Da was not detected at all when peptide **11** was used as the bait in the pull-down experiment. Whereas when the biotinylated TCI **8** is used as the bait, the TVATDGHR peptide from SC was mainly detected with the covalent modification on the histidine residue, even if a small proportion of the peptide remains non-modified (≈12%).

Figure 4 displays a Volcano plot representation showing the distribution of the enriched proteins when comparing either peptide **8** or **11** to the scrambled peptide **10.** The *^{Ec}*SC protein was highly enriched in both cases (log2FC > 9), with very significant adjusted p-values (adjp < 5 10⁻¹⁷). In the pull-down mediated by **8**, three additional proteins (i.e. NagC, TrxA and MoaB) were detected but with a much lower significance (4 10-3 < adjp < 1 10-2) compared to EcSC, probably due to their very low level of detection (average of five spectra for NagC/TrxA/MoaB, average of 129 spectra for *^{Ec}*SC). At best, these proteins are 15 times less enriched than *^{Ec}*SC denoting an excellent selectivity. In the pull-down experiment with **8**, three database search algorithms (Mascot, Sequest and MS-Amanda) successfully identified the covalently his-modified *^{Ec}*SC peptide TVATDGH*R, through y fragment ions and b fragment ions resulting and confirmed covalent bond formation at His175. In addition, the presence of chlorine atoms, from **8** (3,4-di-CI-Phe), in the modified peptide was confirmed by the distortion of its isotopic pattern. The proportion of the covalently modified TVATDGHR peptide compared to non-modified sequence was estimated by an accurate MS1 label-free quantification to be ≈ 86 %.

### The chloroacetamide-based TCI shows superior inhibitory potency over non-covalent inhibitors in vitro

Then, to determine the gain in potency of **8** as compared to the non-covalent inhibitor **11**, the inventors determined the efficiency of each compound to inhibit the processive *E*. *coli* Pollll holoenzyme mediated DNA synthesis in a concentration-dependent DNA synthesis inhibition (Fig. 5)(Wolff, P.; Oliéric, V.; Briand, J. P.; Chaloin, O.; Dejaegere, A.; Dumas, P.; Ennifar, E.; Guichard, G.; Wagner, J.; Burnouf, D. Y. Structure-Based Design of Short Peptide Ligands Binding onto the E. Coli Processivity Ring. J. Med. Chem. 2011, 54 (13), 4627-4637). IC₅₀ were determined after pre-incubating **8** or **11** with *^{Ec}*SC for 10, 90 or 180 min before the addition of the DNA substrate and Pollll. Remarkably, while a little variation of IC₅₀ values was observed with compound **11** between experiments, the IC₅₀ of TCI **8** decreased significantly as a function of time, reaching a 3-fold reduction in IC₅₀ after 180 min. pre-incubation with the target. This undoubtedly results from the time-dependent formation of the covalent adduct. Importantly, this also validates the TCI approach to the well-conserved His residue of bacterial SC to maximize the potency of SC binding antimicrobial agents.

### Conclusion

In summary, we report an effective chloroacetamide-based SC covalent peptide inhibitor targeting a histidine residue. This residue is ideally located at the central part of the SC binding site. Thermodynamic and kinetic studies enabled to select chloroacetamide and acrylamide as the most effective electrophiles to react with His175. X-ray structures of *^{Ec}*SC-TCl adducts were solved at high resolution (1.21 and 1.37,Å) unambiguously demonstrating the formation of the covalent bond with His175 imidazole. Pull-down experiments combined with proteomic analysis showed the high selectivity of the covalent bond formation with the chloroacetamide warhead. Finally, competition experiments with a non-covalent ligand and *in vitro* DNA synthesis inhibition assays indicated the superior potency of TCI to inhibit the bacterial replicative machinery versus non-covalent inhibitors. This study validates the chloroacetamide warhead as an effective and selective electrophile to target a histidine residue.

## Claims

1. A compound having the following formula (I): 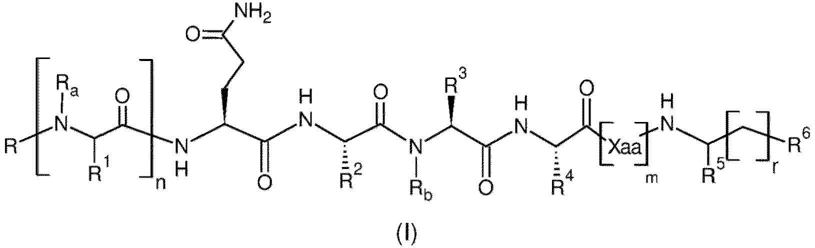 wherein:
- n is 0 or 1;
- m is 0 or 1;
- r is 0 or 1;
- R is H or is selected from the group consisting of:
∗ a (C₁-C₁₈)alkyl group optionally substituted by a (C₆-C₁₀)aryl group, by a heteroaryl group or by a heterocycloalkyl group,
∗ a (C₂-C₁₈)alkenyl group optionally substituted by a (C₆-C₁₀)aryl group,
∗ a (C₃-C₆)cycloalkyl group,
∗ a (C₆-C₁₀)aryl group optionally substituted by a (C₁-C₄)alkyl,
∗ a group having the following formula (II): 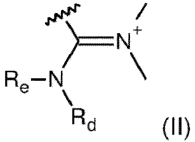 R_{d} and Rₑ representing independently from each other a (C₁-C₆)alkyl group, or forming together with the nitrogen atom carrying them a heterocycloalkyl group,
∗ a group -C(=O)-R', R' being selected from the group consisting of:
· a (C₁-C₁₈)alkyl group optionally substituted by an optionally substituted (C₆-C₁₀)aryl group,
· a -NH-optionally substituted (C₆-C₁₀)aryl group,
· a (C₃-C₆)cycloalkyl group,
· a (C₆-C₁₀)aryl group optionally substituted by a (C₁-C₄)alkyl,
- Rₐ, and R_{b} are, independently from each other, selected from the group consisting of: H, and (C₁-C₆)alkyl groups;
- R¹ is H or the side chain of arginine;
- R² is a (C₁-C₆)alkyl group or a -CH₂-(C₃-C₆)cycloalkyl group;
- R³ is selected from the group consisting of one of the following formulae: 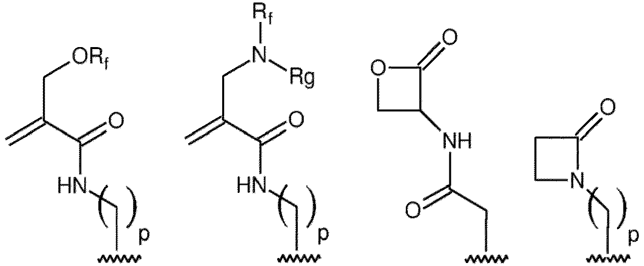 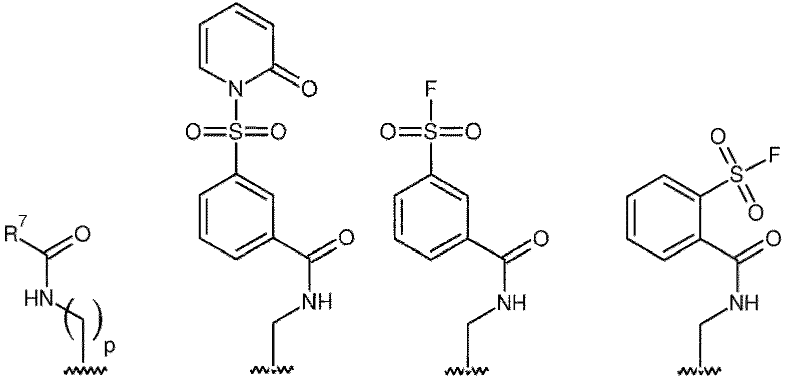 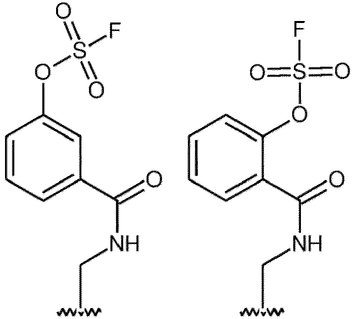 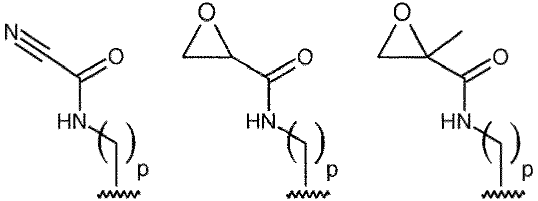 wherein:
· p is 1, 2 or 3;
· R_{f} is selected from the group consisting of: (C₁-C₄)alkyl, (C₆-C₁₀)aryl, acetyl, and COORₕ groups, Rₕ being (C₁-C₄)alkyl;
· R_{g} is selected from the group consisting of: (C₁-C₄)alkyl and (C₆-C₁₀)aryl groups;
· R⁷ is selected from the group consisting of: -CH₂-X, X being Cl or Br, -CH=CH₂,
-CH=CH-CH₃, -C≡CH, -C≡C-CH₃, -CH=CH-CH₂-NMe₂, and -CH₂-O-SO₂-NR_{f}R_{g},
- R⁴ is a (C₁-C₈)alkyl group or a (C₁-C₄)alkyl group optionally substituted by at least one halogen or a -CH₂-(C₃-C₆)cycloalkyl group;
- Xaa is selected from the group consisting of:
· a -N(R_{c})-CH₂-C(=O)- group;
· a group having the following formula (II-1): 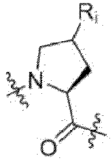
· a group having the following formula (II-2): 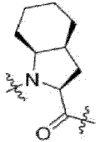 wherein
R_{c} is H or a (C₁-C₆)alkyl group; and
Rᵢ is selected from the group consisting of: H, methyl, OH, F, (C₁-C₄)alkoxy group, (C₁-C₄)alkylthio group, -O-benzyl or -S-benzyl;
- R⁵ is selected in the group consisting of:
∗ a -(CH₂)-(C₆-C₁₀)aryl group optionally substituted by at least one halogen, (C₁-C₂)alkyl group and/or (C₁-C₂)alkoxy group;
∗ a -(CH₂-CH₂)-(C₆-C₁₀)aryl group optionally substituted by at least one halogen, (C₁-C₂)alkyl group and/or (C₁-C₂)alkoxy group;
∗ a -(CH₂)-(C₃-C₆)cycloalkyl group;
∗ a -(CH₂-CH₂)-(C₃-C₆)cycloalkyl group;
∗ a (C₁-C₈)alkyl group, optionally substituted by at least one halogen; and
- R⁶ is H, -COOH, -CONH₂, -COOR¹⁰, or -CONHR¹⁰, R¹⁰ being a (C₁-C₈)alkyl group, when r=0, and R⁶ is H, -COOH, - CONH₂, -OH, -NH₂, -COOR¹⁰, -CONHR¹⁰, or -OR¹⁰, when r=1.

2. The compound of claim 1, wherein R is selected in the group consisting of:
∗ a (C₁-C₁₈)alkyl group optionally substituted by a (C₆-C₁₀)aryl group, by a heteroaryl group or by a heterocycloalkyl group,
∗ a group having the following formula (II): 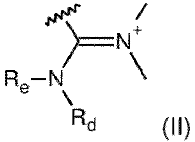 R_{d} and Rₑ representing independently from each other a (C₁-C₆)alkyl group, or forming together with the nitrogen atom carrying them a heterocycloalkyl group, and
∗ a group -C(=O)-R', R' being selected in the group consisting of:
· a (C₁-C₁₈)alkyl group optionally substituted by an optionally substituted (C₆-C₁₀)aryl group, and
· a -NH-optionally substituted (C₆-C₁₀)aryl group.

3. The compound of any one of claim 1 or 2, wherein R² is a -CH₂-(C₃-C₆)cycloalkyl group, preferably a -CH₂-cyclohexyl group.

4. The compound of any one of claims 1 to 3, wherein R³ is selected from the group consisting of one of the following formulae: 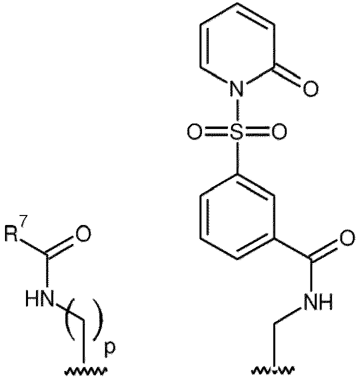 wherein:
. p is 1, 2 or 3; and
. R⁷ is selected from the group consisting of: -CH₂-X, X being Cl or Br, -CH=CH₂, -CH=CH-CH₃, and -C=C-CH₃.

5. The compound of any one of claims 1 to 4, wherein R⁴ is a (C₁-C₈)alkyl group.

6. The compound of any one of claims 1 to 5, wherein R⁵ is a -(CH₂)-(C₆-C₁₀)aryl group optionally substituted by at least one halogen, (C₁-C₂)alkyl group and/or (C₁-C₂)alkoxy group, or a (C₁-C₈)alkyl group or a (C₁-C₄)alkyl group optionally substituted by at least one halogen.

7. The compound of any one of claims 1 to 6, wherein R⁶ is H, -COOH, -CONH₂, or -CO₂R¹⁰, R¹⁰ being a (C₁-C₈)alkyl group, preferably Me, when r=0; and R⁶ is H, -COOH, -CONH₂, -OH, -NH₂, or -CO₂R¹⁰, R¹⁰ being a (C₁-C₈)alkyl group, preferably Me, when r=1.

8. The compound of any one of claims 1 to 7, having one of the following formulae:

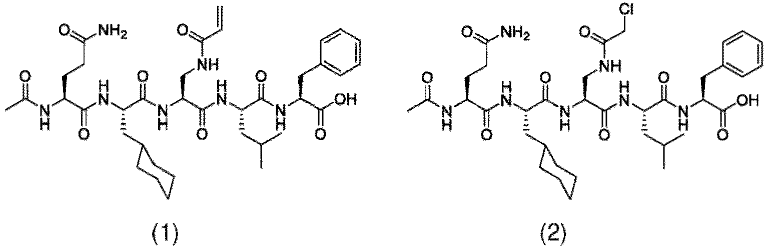

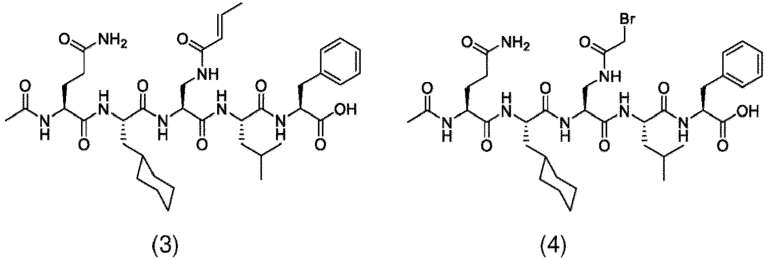

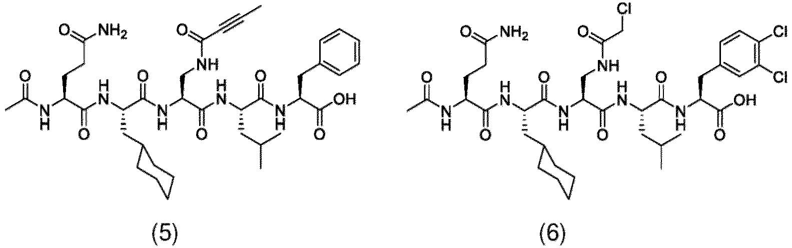

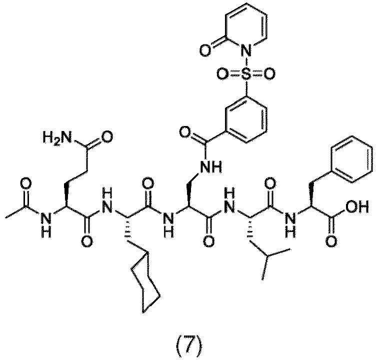

9. A medicament comprising a compound of any one of claims 1 to 8.

10. The compound of any one of claims 1 to 8 for use for the treatment of bacterial infections.
